# EUROPEAN PATENT SPECIFICATION

(11) **EP 3 509 603 B1**
(45) Date of publication and mention of the grant of the patent: **03.01.2024**
(21) Application number: 17794051.7
(22) Date of filing: 07.09.2017
(51) Int. Cl.: A61K 31/575, A61K 31/282, A61P 35/00, A61K 33/24, A61K 33/243, A61P 35/04, C07F 15/00, C07H 23/00, C07J 41/00, C07J 51/00

(54) **IMMUNE MEMORY INDUCTION BY PLATINUM BASED COMPOUNDS**
IMMUNGEDÄCHTNISANREGUNG DURCH PLATINBASIERTE VERBINDUNGEN
INDUCTION DE MÉMOIRE IMMUNITAIRE PAR DES COMPOSÉS À BASE DE PLATINE

(30) Priority: 07.09.2016 IN 201611030627
(43) Date of publication of application: 17.07.2019
(73) Proprietor: Alyssum Therapeutics, Inc., Cambridge, MA 02139 (US)
(72) Inventor: SENGUPTA, Shiladitya, Philadelphia, PA 19104 (US); SENGUPTA, Aniruddha, Delhi 110092 (IN); MYLAVARAPU, Sanghamitra, Delhi 110092 (IN); ROY, Monideepa, Andover, MA 01810 (US)
(74) Representative: Carpmaels & Ransford LLP
(86) International application number: PCT/IB2017/055394
(87) International publication number: WO 2018/047090

(56) References cited:
- WO-A1-2015/153345
- WO-A2-03/095462
- WO-A2-2010/091192
- WO-A2-2014/201376
- CN-B- 104 610 415

## Description

### TECHNICAL FIELD

The present invention is in the field of pharmaceutical sciences and medicinal chemistry. The present invention relates to method of treating or managing cancer and preventing cancer metastasis or relapse by employing platinum based compound(s) which are capable of modulating host immune system and increasing tumor-infiltration of immune cells, leading to altered expression of immune markers. Said compound(s) are useful in immunotherapy as they induce immune memory in the host immune system.

### BACKGROUND AND PRIOR ART OF THE INVENTION

Cancer is a disease involving uncontrolled growth of cells. It is a clinically complex disease, where multiple parameters, including the tumor microenvironment and immune response in the patient contribute to disease progression as well as selection and outcome of therapy. Though tumor have numerous antigens, which can be recognized by the immune system, the tumor's ability to escape the immune system or suppress it often makes the immune mechanism insufficient to prevent tumor growth. Cancer is treated using a variety of modalities including surgery, radiation therapy, chemotherapy, targeted therapy, which includes immunotherapy. Immunotherapies have high specificity and can reduce side effects, associated with most chemotherapies and can be implemented to improve the patient's quality of life.

Combinatorial treatments with chemotherapeutics and immunotherapies are currently being investigated in several cancers. However, a tumor is a complex milieu of cancer cells, extracellular matrix components, supportive stromal cells and a number of inflammatory cells. There are complexities involved in mounting an anti-tumor immune response, as the priming occurs in lymph nodes and effector functions operate in the tumor mass. In addition, barriers to anti-tumor responses, including lack of "signals" from innate immune cells, poor recruitment of Dendritic Cells, inadequate expression of costimulatory ligands on tumor cells or antigen presenting cells (APCs) influence the immune response. (Harris and Drake, Journal for Immuno Therapy of Cancer 2013 1:12). These approaches have mostly been tried when conventional therapies have failed, to significantly increase survival in patients and have given a better understanding on how tolerance, immunity and immunosuppression regulate antitumor immune responses (Mellman et al., Nature, 2011, Vol 480; 480-489). These success stories initiated studies to understand the immunomodulatory effects of clinically approved cytotoxic drugs, including platinates. These effects are important in combating tumors, as emerging antineoplastic strategies are increasingly engaging the immune system directly (e.g., checkpoint blockade and adoptive T-cell therapies), with a goal of achieving synergy in the process.

Within the last two decades, activation of the immune system has been evaluated as a therapeutic approach to mediate anti-tumor activity. Generally, a host response to tumor cells begins with T-cell recognition of tumor associated antigens on tumor cells or via antigen presenting cells. Recognition via T-cell antigen receptor triggers signal transduction pathways that mediate activation of the T-cell. This results in secretion of interleukin-2 (IL-2), gamma-interferon (INF-γ), tumor necrosis factor-alpha (TNF-α), and other cytokines from the T-cells and accessory cells, which mobilizes the host immune system to kill tumor cells. In addition to the T-cell receptor and MHC antigens, numerous cell surface antigens have been identified, which play crucial role in mediating interactions between antigen presenting cells and the responder T-cells (Pardi et al., Immunol. Today 13, p. 224-230 (1992); Chen et al., Immunol. Today 14, p. 483-486 (1993)).

In addition, while B cells have long been known to produce antibodies, their ability to act as effector cells in an immune response has been recognized relatively recently (Harris et al. (2000), Nat Immunol 1:475-482; Li et al. (2009) J Immunol 183:3195-3203). The following emerging research findings indicate that: (1) B cells have a major impact on tumorigenesis; (2) targeting B cells may improve the efficacy of T-cell-mediated immunotherapy, and (3) B cells themselves may have important antitumor activity in some settings. It is interesting to note that in medullary breast cancer, a favorable prognosis is associated with infiltrates of B cells and plasma cells (Hansen et al. (2001), Proc Natl Acad Sci USA 98:12659-12664). Tumorinfiltrating B cells (TIBs) are also found in other types of breast cancer (Pavoni et al. (2007), BMC Biotechnol 7:70) and other cancers including melanoma (Zhang et al (1995), Cancer Res 55:3584-3591), lung cancer (Imahayashi et al (2000), Cancer Invest 18:530-536) and mesothelioma (Shigematsu et al (2009), Cancer Sci 100:1326-1334).

Chemotherapeutics can increase the immunogenicity of tumors besides modulating the immune system. Platinates have been shown to (1) upregulate MHC class I expression; (2) promote recruitment and proliferation of effector cells and (3) downregulate immunosuppressive microenvironment (de Biasi et al., Clin Cancer Res. 2014;20:5384-91). Experiments in immunocompetent versus immunodeficient mice demonstrated that some chemotherapeutic compounds, including oxaliplatin, are more effective in the presence of an intact immune system and can induce tumor-specific immune responses (Apetoh et al., Nat Med 2007;13:1050-9.35-37; Tesniere et al., Oncogene 2010;29:482-91).

Platinum drugs have been shown to modulate host immune system by altering the expression of immune markers and increase tumor immunogenicity by facilitating tumor-infiltration of immune cells. Oxaliplatin in particular, demonstrates a tumor-specific immune response and is a potent stimulator of immunogenic cell death (Tesniere et al. (2010), Oncogene. 29(4):482-91). Denkert et. al evaluated the tumor infiltrating immune cells and measured the relative mRNA expression levels of immune activating and immune suppressive genes upon combinatorial treatment regimen containing carboplatin. Their results indicate significant predictive value of infiltrating immune cells and expression levels of immunologically relevant genes towards therapy outcome (Clin Oncol. 2015 Mar 20;33(9):983-91). However, there is still need for methods which can more effectively treat tumor or cancer and prevent cancer metastasis or relapse and still a need for potent compounds, which can help in achieving long lasting effects in cancer therapeutics.

In view of the above, the present invention seeks to provide more effective methods of treating cancer or tumor, and more importantly to combat relapse of a cancer or tumor or metastasis in a subject, by employing novel compounds, for which methods have not been disclosed yet in the art. The mechanism of stimulating immune response in a host/subject, by novel platinum compounds, by inducing immune memory underlying the treatment module in the present invention has been decoded for the first time and thereby is of significance. The compound(s) and method(s) provided by the present invention, provide solutions to the problems existing currently in the field of cancer therapy and diagnostics.

### SUMMARY OF THE INVENTION

The present invention provides a compound of Formula I or II, or a salt thereof, for use in a method of inducing immune memory in a subject having cancer, said method comprising administering an amount of a compound of Formula I or Formula II, or a salt thereof, to the subj ect. 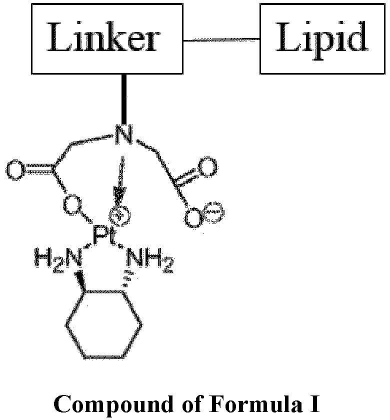 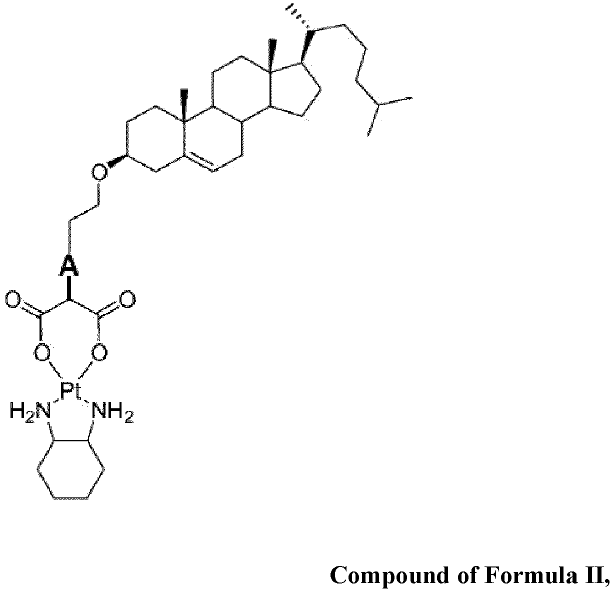 Wherein:
'A' is optionally present and wherein 'A' is cyclobutyl;
the lipid is a sterol; and
the linker is -CH₂CH₂-, -CH₂CH₂NHC(O)-, -CH₂C(O)NHCH₂CH₂-, - CH₂CH₂OCH₂CH₂₋ -C(O)CH₂-, or -CH₂CH₂NHC(O)CH₂-.

In an embodiment, the compound is

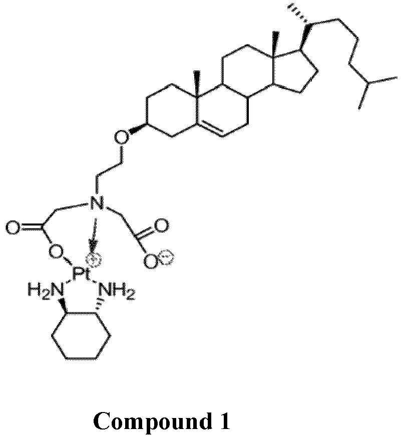

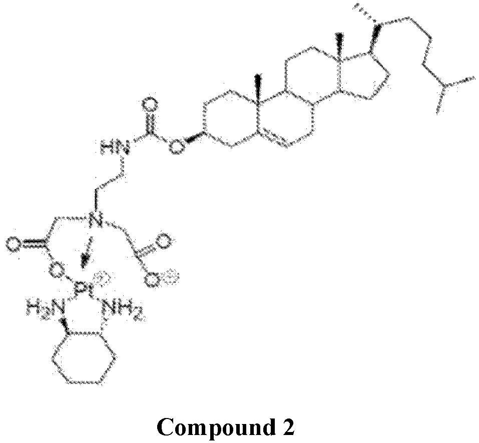

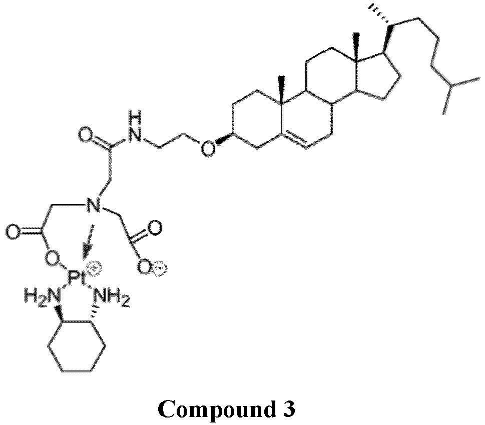

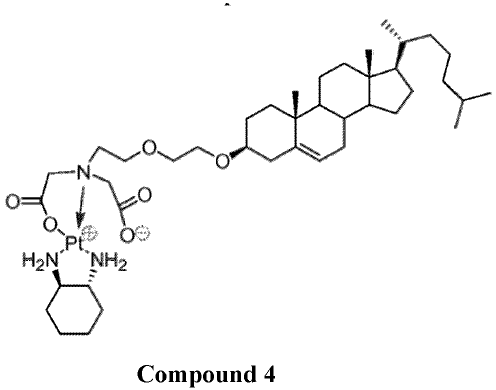

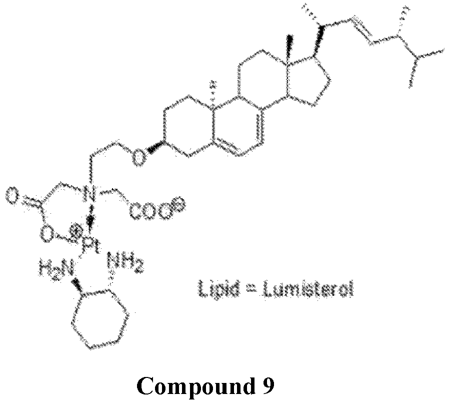

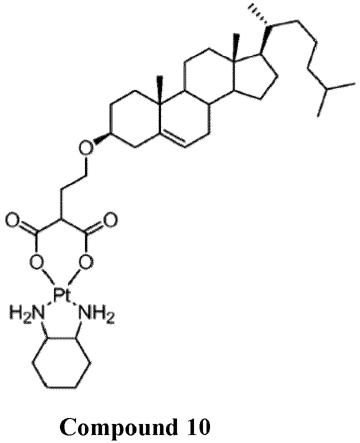

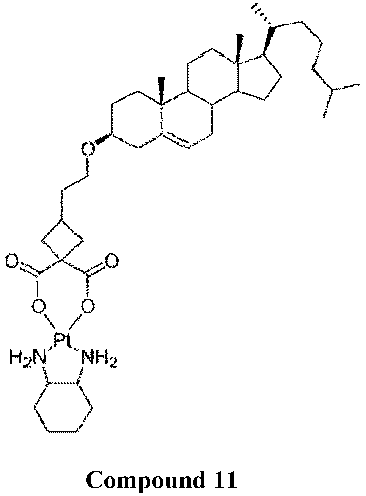

In another embodiment, the subject is a mammal, including human.

In another embodiment, the cancer is selected from a group consisting of breast cancer, ovarian cancer, glioma, gastrointestinal cancer, prostate cancer, carcinoma, lung carcinoma, hepatocellular carcinoma, testicular cancer, cervical cancer, endometrial cancer, bladder cancer, head and neck cancer, lung cancer, gastro-esophageal cancer and gynecological cancer, or any combination thereof.

In another embodiment, the compound of Formula I or Formula II is its derivative, salt form, tautomeric form, isomer, polymorph, solvate and intermediates thereof.

The lipid moiety in the compound of Formula I is a sterol, preferably a sterol selected from lumisterol, cholesterol, cholesterol chloroformate and derivatives thereof, or any combination thereof.

The linker in the compound of Formula I is -CH₂CH₂-, - CH₂CH₂NHC(O)-, - CH₂C(O)NHCH₂CH₂-, -CH₂CH₂OCH₂CH₂-, -C(O)CH₂-, or - CH₂CH₂NHC(O)CH₂-.

In another embodiment, the compound of Formula I or Formula II is administered at dosage where the platinum concentration ranges from about 50 mg/m² to about 500 mg/m².

In another embodiment, the compound of Formula I or Formula II is administered via intravenous administration, intra articular administration, pancreatic duodenal artery administration, intraperitoneal administration, oral administration, hepatoportal administration or intramuscular administration; optionally along with pharmaceutically acceptable excipient(s).

In another embodiment, the excipient(s) is selected from a group consisting of granulating agents, binding agents, lubricating agents, disintegrating agents, sweetening agents, glidants, anti-adherents, anti-static agents, surfactants, anti-oxidants, gums, coating agents, coloring agents, flavouring agents, coating agents, plasticizers, preservatives, suspending agents, emulsifying agents, plant cellulosic material and spheronization agents, or any combination thereof.

In another embodiment, the compound of Formula I or Formula II is formulated into a dosage form selected from a group consisting of injectable, tablet, lyophilized powder and liposomal suspension, or any combination thereof.

In another embodiment, the compound of Formula I or Formula II enhances expression of immunoglobulin kappa C in tumor microenvironment of the cancer subject.

In another embodiment, the compound of Formula I or Formula II prevents the metastasis or the relapse by induction of immune response mediated through immunopotentiating molecule(s), which thereby activate cytokine(s), B-cell(s), T-cell(s), monocyte(s), macrophage(s), Natural Killer cell(s), dendritic cell(s) or a combination thereof.

In another embodiment, the compound of Formula I or Formula II prevents metastasis or the relapse by triggering humoral immune response through B cell(s); and wherein the B-cell(s) is selected from a group consisting of Plasmablast, Plasma cell, Lymphoplasmacytoid cell, Memory B cell, Follicular B cell, Marginal zone B cell, B-1 cell, B-2 cell and Regulatory B cell, or any combination thereof.

In another embodiment, the T-cell(s) is selected from a group consisting of T helper cells, Cytotoxic T cells, memory T cells, suppressor T cells, Natural killer T cells, Mucosal associated invariant T cells and Gamma delta T cells, or any combination thereof.

In another embodiment, the immune response is activated via nucleic acid adduct formation, preferably via a double-stranded DNA adduct, single-stranded DNA adduct, double-stranded RNA adduct, or single-stranded RNA adduct.

The present invention also relates to a compound of Formula I or II, or a salt thereof, for use in a method of inducing immune memory in a subject have cancer, wherein said method is for treating or managing cancer and preventing metastasis or relapse of the cancer in a subject and comprises administering to said subject a therapeutically effective amount of said compound of Formula I or Formula II. The compound 1 is the preferred compound employed from the group of compounds depicted or encompassed by compound of formula I.

The present invention also relates to a compound of Formula I or II, for use in a method of inducing immune memory in a subject having cancer, said method enhancing immune response of a subject suffering from cancer, wherein said method comprises treating the cancer with a therapeutically effective amount of compound of Formula I or Formula II, preferably by Compound 1.

### BRIEF DESCRIPTION OF THE ACCOMPANYING FIGURES

In order that the invention may be readily understood and put into practical effect, reference will now be made to exemplary embodiments as illustrated with reference to the accompanying figures. The figures together with a detailed description below, are incorporated in and form part of the specification, and serve to further illustrate the embodiments and explain various principles and advantages, in accordance with the present invention.
Fig. 1. (A) Examination of tumor infiltrating immune cells by evaluating their relative mRNA profile in treated tumors. (B) Immunohistochemical analysis of markers of B cell lineage in treated tumors. (*, P ≤ 0.05)
**Fig. 2**. (A) Activation of TCR by Compound of Formula I. (B) Infiltration of cytotoxic CD8+ T-cells in tumors treated with Compound of Formula I.
**Fig. 3**. (A) Schematic representation of study to evaluate immune memory. (B) Compound of Formula I regresses tumor in a murine TNBC model. (C) Compound of Formula I induces immune memory only in tumor bearing animals. Arrows depict injection of cells, while arrowheads indicate dosing of Compound of Formula I.
**Fig. 4****.** Compound of Formula I shows tumor regression only in immunocompetent mice.
Tumors do not regress in mice lacking immune cells. Arrows indicate dosing of Compound of Formula I.
**Fig. 5****.** Cellular imaging depicting Propidium iodide localization in platinate treated cells under (A) low and (B) high magnification. (C) Relative Fluorescence measurement per field in DNase and RNase treated samples. (**, P ≤ 0.005).
**Fig. 6**. (A) Schematic representation to study splenic B-cells. (B) Examination of Plasma B cell differentiation and TLR activation markers by evaluating their relative mRNA profile in treated tumors. All values normalized to splenic B cells isolated from group 1 mice.
**Fig. 7**. (A) Schematic representation to study the role of 4T1 conditioned media in TLR activation and differentiation of splenic B-cells. (B) Examination of Plasma B cell differentiation and TLR activation markers by evaluating their relative mRNA profile in B-cells treated with conditioned media from 4T1 cells. (C) Examination of Plasma B cell differentiation and TLR activation markers by evaluating their relative mRNA profile in B-cells treated with nucleic acid depleted conditioned media from 4T1 cells. All values normalized to splenic B cells treated with 4T1 conditioned media.

### DETAILED DESCRIPTION OF THE INVENTION

Unless otherwise defined herein, scientific and technical terms used in connection with the present invention shall have the meanings that are commonly understood by those of ordinary skill in the art. Further, unless otherwise required by context, singular terms shall include the plural and plural terms shall include the singular as is considered appropriate to the context and/or application. The various singular/plural permutations may be expressly set forth herein for the sake of clarity. Generally, nomenclatures used in connection with, pharmaceutical sciences and chemical industry described herein are those well-known and commonly used in the art. In case of conflict, the present specification, including definitions, will control. The materials, methods, figures and examples are illustrative only and not intended to be limiting.

It is to be understood that the terminologies used herein are for the purpose of describing particular embodiments only and are not intended to be limiting. It must be noted that, as used in the specification and the appended claims, the singular forms "a," "an" and "the" include plural referents unless the context clearly dictates otherwise.

As used herein, the terms tumor and cancer are used interchangeably and reference of treating either is to be considered as appropriate treatment for both. A tumor or cancer in the present invention are encompassed to possess malignant cell/tissues.

As used herein, the term "immunopotentiating molecule(s)" refers to, but is not limited to any of a wide variety of specific or nonspecific substances that on administration involves stimulation of biologic molecules and complexes, or cellular, cell, or tissue components of a normal immune response.

As used herein, the term "nucleic acid adduct" refers to, a chemical agent bound to a segment of nucleic acid (RNA or DNA).

As used herein, the term "immune memory" refers to ability of the immune system to remember antigens that it encountered previously and respond faster with higher efficacy when encountering the same antigens again.

As used herein, the term "lipid" is used in the conventional sense and includes compounds of varying chain length, from as short as about 2 carbon atoms to as long as about 28 carbon atoms. Additionally, the compounds may be saturated or unsaturated and in the form of straight-or branched-chains or in the form of unfused or fused ring structures. Exemplary lipids include but are not limited to fats, waxes, sterols, steroids, bile acids, fat-soluble vitamins (such as A, D, E and K), monoglycerides, diglycerides, phospholipids, glycolipids, sulpholipids, aminolipids, chromolipids (lipochromes), glycerophospholipids, sphingolipids, prenollipids, saccharolipids, polyketides, and fatty acids.

In addition to the platinum compounds disclosed herein, the particle can comprise co-lipids and/stabilizers. Additional lipids can be included in the particles for a variety of purposes, such as to prevent lipid oxidation, to stabilize the bilayer, to reduce aggregation during formation or to attach ligands onto the particle surface. Any of a number of additional lipids and/or other components can be present, including amphipathic, neutral, cationic, anionic lipids, and programmable fusion lipids. Such lipids and/or components can be used alone or in combination. One or more components of particle can comprise a ligand, e.g., a targeting ligand.

In some embodiments, the particle further comprises a phospholipid. Without limitations, the phospholipids can be of natural origin, such as egg yolk or soybean phospholipids, or synthetic or semisynthetic origin. The phospholipids can be partially purified or fractionated to comprise pure fractions or mixtures of phosphatidyl cholines, phosphatidyl cholines with defined acyl groups having 6 to 22 carbon atoms, phosphatidyl ethanolamines, phosphatidyl inositols, phosphatidic acids, phosphatidyl serines, sphingomyelin or phosphatidyl glycerols. Suitable phospholipids include, but are not limited to, phosphatidylcholine, phosphatidylglycerol, lecithin, β,γ-dipalmitoyl-α-lecithin, sphingomyelin, phosphatidylserine, phosphatidic acid, N-(2,3-di(9-(Z)-octadecenyloxy))-prop-1-yl-N,N,N-trimethylammonium chloride, phosphatidylethanolamine, lysolecithin, lysophosphatidylethanolamine, phosphatidylinositol, cephalin, cardiolipin, cerebrosides, dicetylphosphate, dioleoylphosphatidylcholine, dipalmitoylphosphatidylcholine, dipalmitoylphosphatidylglycerol, dioleoylphosphatidylglycerol, palmitoyl-oleoyl-phosphatidylcholine, di-stearoyl-phosphatidylcholine, stearoyl-palmitoyl-phosphatidylcholine, di-palmitoyl-phosphatidylethanolamine, di-stearoyl-phosphatidylethanolamine, di-myrstoyl-phosphatidylserine, di-oleyl-phosphatidylcholine, dimyristoyl phosphatidyl choline (DMPC), dioleoylphosphatidylethanolamine (DOPE), palmitoyloleoylphosphatidylcholine (POPC), egg phosphatidylcholine (EPC), distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), - phosphatidylethanolamine (POPE), dioleoyl-phosphatidylethanolamine 4-(N-maleimidomethyl)-cyclohexane-1-carboxylate (DOPE-mal), 1-stearoyl-2-oleoyl phosphatidylcholine (SOPC), 1,2-distearoyl-sn-glycem-3-phosphoethanolamine (DSPE), and any combinations thereof. Non-phosphorus containing lipids can also be used. These include, e.g., stearylamine, docecylamine, acetyl palmitate, fatty acid amides, and the like. Other phosphorus-lacking compounds, such as sphingolipids, glycosphingolipid families, diacylglycerols, and β-acyloxyacids, can also be used

In some embodiments, the phospholipid in the particle is selected from the group consisting of 1,2-Didecanoyl-*sn*-glycero-3-phosphocholine; 1,2-Dierucoyl-*sn*-glycero-3-phosphate (Sodium Salt); 1,2-Dierucoyl-*sn*-glycero-3-phosphocholine; 1,2-Dierucoyl-*sn*-glycero-3-phosphoethanolamine; 1,2-Dierucoyl-*sn*-glycero-3[Phospho-rac-(1-glycerol) (Sodium Salt); 1,2-Dilinoleoyl-*sn*-glycero-3-phosphocholine; 1,2-Dilauroyl-*sn*-glycero-3-phosphate (Sodium Salt); 1,2-Dilauroyl-*sn*-glycero-3-phosphocholine; 1,2-Dilauroyl-*sn*-glycero-3-phosphoethanolamine; 1,2-Dilauroyl-*sn*-glycero-3 [Phospho-rac-(1-glycerol) (Sodium Salt); 1,2-Dilauroyl-*sn*-glycero-3 [Phospho-rac-(1-glycerol) (Ammonium Salt); 1,2-Dilauroyl-*sn-*glycero-3-phosphoserine (Sodium Salt); 1,2-Dimyristoyl-*sn*-glycero-3-phosphate (Sodium Salt); 1,2-Dimyristoyl-*sn*-glycero-3-phosphocholine; 1,2- Dimyristoyl-*sn*-glycero-3-phosphoethanolamine; 1,2-Dimyristoyl-*sn*-glycero-3[Phospho-rac-(1-glycerol) (Sodium Salt); 1,2-Dimyristoyl-*sn*-glycero-3[Phospho-rac-(1-glycerol) (Ammonium Salt); 1,2-Dimyristoyl-*sn*-glycero-3 [Phospho-rac-(1-glycerol) (Sodium/Ammonium Salt); 1,2-Dimyristoyl-*sn-*glycero-3-phosphoserine (Sodium Salt); 1,2-Dioleoyl-*sn*-glycero-3-phosphate (Sodium Salt); 1,2-Dioleoyl-*sn*-glycero-3-phosphocholine; 1,2-Dioleoyl-*sn*-glycero-3-phosphoethanolamine; 1,2-Dioleoyl-*sn*-glycero-3 [Phospho-rac-(1-glycerol) (Sodium Salt); 1,2-Dioleoyl-*sn*-glycero-3-phosphoserine (Sodium Salt); 1,2-Dipalmitoyl-*sn*-glycero-3-phosphate (Sodium Salt); 1,2-Dipalmitoyl-*sn*-glycero-3-phosphocholine; 1,2-Dipalmitoyl-*sn*-glycero-3-phosphoethanolamine; 1,2-Dipalmitoyl-*sn*-glycero-3[Phospho-rac-(1-glycerol) (Sodium Salt); 1,2-Dipalmitoyl-*sn*-glycero-3[Phospho-rac-(1-glycerol) (Ammonium Salt); 1,2-Dipalmitoyl-*sn*-glycero-3-phosphoserine (Sodium Salt); 1,2-Distearoyl-*sn*-glycero-3-phosphate (Sodium Salt); 1,2-Distearoyl-*sn*-glycero-3-phosphocholine; 1,2-Distearoyl-*sn*-glycero-3-phosphoethanolamine; 1,2-Distearoyl-*sn*-glycero-3[Phospho-rac-(1-glycerol) (Sodium Salt); 1,2-Distearoyl-*sn*-glycero-3[Phospho-rac-(1-glycerol) (Ammonium Salt); 1,2-Distearoyl-*sn-*glycero-3-phosphoserine (Sodium Salt); Egg-PC; Hydrogenated Egg PC; Hydrogenated Soy PC; 1-Myristoyl-*sn*-glycero-3-phosphocholine; 1-Palmitoyl-*sn*-glycero-3-phosphocholine; 1-Stearoyl-*sn*-glycero-3-phosphocholine; 1-Myristoyl-2-palmitoyl-*sn*-glycero 3-phosphocholine; 1-Myristoyl-2-stearoyl-*sn*-glycero-3-phosphocholine; 1-Palmitoyl-2-myristoyl-*sn*-glycero-3-phosphocholine; 1-Palmitoyl-2-oleoyl-*sn*-glycero-3-phosphocholine; 1-Palmitoyl-2-oleoyl-*sn*-glycero-3-phosphoethanolamine; 1-Palmitoyl-2-oleoyl-*sn*-glycero-3[Phospho-rac-(1-glycerol)] (Sodium Salt); 1-Palmitoyl-2-stearoyl-*sn*-glycero-3-phosphocholine; 1-Stearoyl-2-myristoyl-*sn*-glycero-3-phosphocholine; 1-Stearoyl-2-oleoyl-*sn*-glycero-3-phosphocholine; and 1-Stearoyl-2-palmitoyl-*sn*-glycero-3-phosphocholine. In some embodiments, the phospholipid is SPOC, egg PC, or Hydrogenated Soy PC (HSPC). In one, the phospholipid in the composition is HSPC.

In some embodiments, the particle further comprises a polyethylene glycol (PEG). The PEG can be included in the particle by itself or conjugated with a component present in the particle. For example, the PEG can be conjugated with the platinum based compound or a co-lipid/stabilizer component of the particle. In some embodiments, the PEG is conjugated with a co-lipid component of the particle. Without limitations, the PEG can be conjugated with any co-lipid. For example, the PEG conjugated co-lipid can be selected from the group consisting of PEG conjugated diacylglycerols and dialkylglycerols, PEG- conjugated phosphatidylethanolamine, PEG conjugated to phosphatidic acid, PEG conjugated ceramides (*see*, U.S. Patent No. 5,885,613), PEG conjugated dialkylamines, PEG conjugated 1,2-diacyloxypropan-3-amines, and PEG conjugated to 1,2-distearoyl-sn-glycem-3-phosphoethanolamine (DSPE), and any combinations thereof. In some embodiments, the PEG conjugated lipid is 1,2-distearoyl-sn-glycem-3-phosphoethanolamine-N-[amino(polyethylene glycol)-2000] (DSPE-PEG2000).

In some embodiments, the particle further comprises a surfactant. Surfactants find wide application in formulations such as emulsions (including microemulsions) and liposomes. The most common way of classifying and ranking the properties of the many different types of surfactants, both natural and synthetic, is by the use of the hydrophile/lipophile balance (HLB). The nature of the hydrophilic group (also known as the "head") provides the most useful means for categorizing the different surfactants used in formulations (Rieger, in Pharmaceutical Dosage Forms, Marcel Dekker, Inc., New York, N.Y., 1988, p. 285).

If the surfactant molecule is not ionized, it is classified as a nonionic surfactant. Nonionic surfactants find wide application in pharmaceutical and cosmetic products and are usable over a wide range of pH values. In general their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The use of surfactants in drug products, formulations and in emulsions has been reviewed (Rieger, in Pharmaceutical Dosage Forms, Marcel Dekker, Inc., New York, N.Y., 1988, p. 285).

In some embodiments, the particle can further comprise acationic lipid. Exemplary cationic lipids include, but are not limited to, N,N-dioleyl-N,N-dimethylammonium chloride (DODAC), N,N-distearyl-N,N-dimethylammonium bromide (DDAB), N-(1-(2,3-dioleoyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2,3-dioleyloxy)propylamine (DODMA), 1,2-DiLinoleyloxy-N,N-dimethylaminopropane (DLinDMA), 1,2-Dilinolenyloxy-N,N-dimethylaminopropane (DLenDMA), 1,2-Dilinoleylcarbamoyloxy-3-dimethylaminopropane (DLin-C-DAP), 1,2-Dilinoleyoxy-3-(dimethylamino)acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S-DMA), 1-Linoleoyl-2-linoleyloxy-3-dimethylaminopropane (DLin-2-DMAP), 1,2-Dilinoleyloxy-3-trimethylaminopropane chloride salt (DLin-TMA.C1), 1,2-Dilinoleoyl-3-trimethylaminopropane chloride salt (DLin-TAP.C1), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DMA), 1,2-Dilinolenyloxy-N,N-dimethylaminopropane (DLinDMA), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech Gi), or a mixture thereof.

In some embodiments, the particle further comprises a non-cationic lipid. The non-cationic lipid can be an anionic lipid or a neutral lipid including, but not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoylphosphatidylethanolamine (POPE), dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1-trans PE, 1-stearoyl-2-oleoyl-phosphatidyethanolamine (SOPE), cholesterol, or a mixture thereof.

The conjugated lipids that inhibits aggregation of particles can also be included in the particles disclosed herein. Such lipids include, but are not limited to, a polyethyleneglycol (PEG)-lipid including, without limitation, a PEG-diacylglycerol (DAG), a PEG-dialkyloxypropyl (DAA), a PEG-phospholipid, a PEG-ceramide (Cer), or a mixture thereof. The PEG-DAA conjugate can be, for example, a PEG-dilauryloxypropyl (C₁₂), a PEG-dimyristyloxypropyl (C₁₄), a PEG-dipalmityloxypropyl (C₁₆), or a PEG-distearyloxypropyl (C₁₈). The conjugated lipid that prevents aggregation of particles can be from 0.01 mol % to about 20 mol % or about 2 mol % of the total lipid present in the particle.

In some embodiments, the particle is in the form of a liposome, vesicle, or emulsion. As used herein, the term "liposome" encompasses any compartment enclosed by a lipid layer. Liposomes can have one or more lipid membranes. Liposomes can be characterized by membrane type and by size. Small unilamellar vesicles (SUVs) have a single membrane and typically range between 0.02 and 0.05 µm in diameter; large unilamellar vesicles (LUVS) are typically larger than 0.05 µm. Oligolamellar large vesicles and multilamellar vesicles have multiple, usually concentric, membrane layers and are typically larger than 0.1 µm. Liposomes with several nonconcentric membranes, i.e., several smaller vesicles contained within a larger vesicle, are termed multivesicular vesicles.

In order to form a liposome the lipid molecules comprise elongated non-polar (hydrophobic) portions and polar (hydrophilic) portions. The hydrophobic and hydrophilic portions of the molecule are preferably positioned at two ends of an elongated molecular structure. When such lipids are dispersed in water they spontaneously form bilayer membranes referred to as lamellae. The lamellae are composed of two mono layer sheets of lipid molecules with their non-polar (hydrophobic) surfaces facing each other and their polar (hydrophilic) surfaces facing the aqueous medium. The membranes formed by the lipids enclose a portion of the aqueous phase in a manner similar to that of a cell membrane enclosing the contents of a cell. Thus, the bilayer of a liposome has similarities to a cell membrane without the protein components present in a cell membrane.

As used herein, the term "linker" means an organic moiety that connects two parts of a compound.

A cleavable linking group is one which is sufficiently stable outside the cell, but which upon entry into a target cell is cleaved to release the two parts the linker is holding together. In a preferred embodiment, the cleavable linking group is cleaved at least 10 times or more, preferably at least 100 times faster in the target cell or under a first reference condition (which can, e.g., be selected to mimic or represent intracellular conditions) than in the blood or serum of a subject, or under a second reference condition (which can, e.g., be selected to mimic or represent conditions found in the blood or serum).

Cleavable linking groups are susceptible to cleavage agents, e.g., pH, redox potential or the presence of degradative molecules. Generally, cleavage agents are more prevalent or found at higher levels or activities inside cells than in serum or blood. Examples of such degradative agents include: redox agents which are selected for particular substrates or which have no substrate specificity, including, e.g., oxidative or reductive enzymes or reductive agents such as mercaptans, present in cells, that can degrade a redox cleavable linking group by reduction; esterases; amidases; endosomes or agents that can create an acidic environment, e.g., those that result in a pH of five or lower; enzymes that can hydrolyze or degrade an acid cleavable linking group by acting as a general acid, peptidases (which can be substrate specific) and proteases, and phosphatases.

A linker can include a cleavable linking group that is cleavable by a particular enzyme. The type of cleavable linking group incorporated into a linker can depend on the cell to be targeted. For example, liver targeting ligands can be linked to the cationic lipids through a linker that includes an ester group. Liver cells are rich in esterases, and therefore the linker will be cleaved more efficiently in liver cells than in cell types that are not esterase-rich. Other cell-types rich in esterases include cells of the lung, renal cortex, and testis. Linkers that contain peptide bonds can be used when targeting cell types rich in peptidases, such as liver cells and synoviocytes. In some embodiments, cleavable linking group is cleaved at least 1.25, 1.5, 1.75, 2, 3, 4, 5, 10, 25, 50, or 100 times faster in the cell (or under in vitro conditions selected to mimic intracellular conditions) as compared to blood or serum (or under in vitro conditions selected to mimic extracellular conditions). In some embodiments, the cleavable linking group is cleaved by less than 90%, 80%, 70%, 60%, 50%, 40%, 30%, 20%, 10%, 5%, or 1% in the blood (or in vitro conditions selected to mimic extracellular conditions) as compared to in the cell (or under in vitro conditions selected to mimic intracellular conditions).

Exemplary cleavable linking groups include, but are not limited to, redox cleavable linking groups (e.g., -S-S- and -C(R)₂-S-S-, wherein R is H or C₁-C₆ alkyl and at least one R is C₁-C₆ alkyl such as CH₃ or CH₂CH₃); phosphate-based cleavable linking groups (e.g., -O-P(O)(OR)-O-, -O-P(S)(OR)-O-, -O-P(S)(SR)-O-, -S-P(O)(OR)-O-, -O-P(O)(OR)-S-, -S-P(O)(OR)-S-, - O-P(S)(ORk)-S-, -S-P(S)(OR)-O-, -O-P(O)(R)-O-, -O-P(S)(R)-O-, -S-P(O)(R)-O-, -S-P(S)(R)-O-, -S-P(O)(R)-S-, -O-P(S)( R)-S-, . -O-P(O)(OH)-O-, -O-P(S)(OH)-O-, -OP(S)(SH)-O-, -S-P(O)(OH)-O-, -O-P(O)(OH)-S-, -S-P(O)(OH)-S-, -O-P(S)(OH)-S-, -S-P(S)(OH)-O-, -O-P(O)(H)-O-, -O-P(S)(H)-O-, -S-P(O)(H)-O-, -S-P(S)(H)-O-, -S-P(O)(H)-S-, and -O-P(S)(H)-S-, wherein R is optionally substituted linear or branched C₁-C₁₀ alkyl); acid celavable linking groups (e.g., hydrazones, esters, and esters of amino acids, -C=NN- and - OC(O)-); ester-based cleavable linking groups (e.g., -C(O)O-); peptide-based cleavable linking groups, (e.g., linking groups that are cleaved by enzymes such as peptidases and proteases in cells, e.g., - NHCHR^{A}C(O)NHCHR^{B}C(O)-, where R^{A} and R^{B} are the R groups of the two adjacent amino acids). A peptide based cleavable linking group comprises two or more amino acids. In some embodiments, the peptide-based cleavage linkage comprises the amino acid sequence that is the substrate for a peptidase or a protease found in cells.

In some embodiments, an acid cleavable linking group is cleavable in an acidic environment with a pH of about 6.5 or lower (e.g., about 6.5, 6.0, 5.5, 5.0, or lower), or by agents such as enzymes that can act as a general acid.

The present invention relates to a compound of Formula I or II, or a salt thereof, for use in a method of inducing immune memory in a subject having cancer, comprising administering an amount of a compound of Formula I or II, or a salt thereof, to the subject. 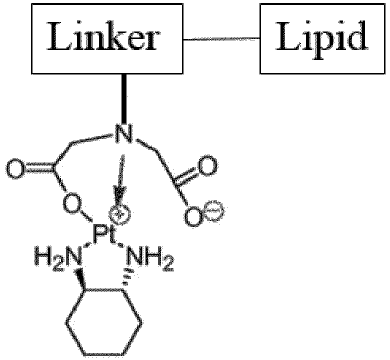 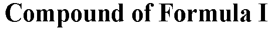 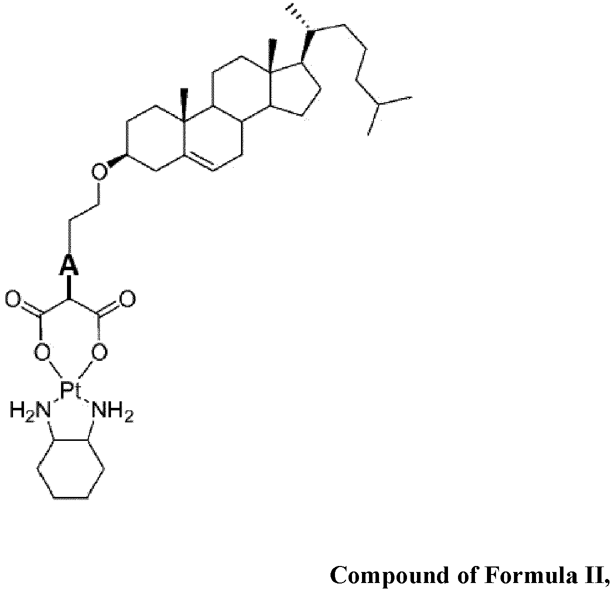 wherein:
'A' is optionally present and wherein 'A' is cyclobutyl;
the lipid is a sterol; and
the linker is -CH₂CH₂-, -CH₂CH₂NHC(O)-, -CH2C(O)NHCH₂CH₂-, - CH₂CH₂OCH₂CH₂-, -C(O)CH₂-, or -CH₂CH₂NHC(O)CH₂-.

In an embodiment, the compound is

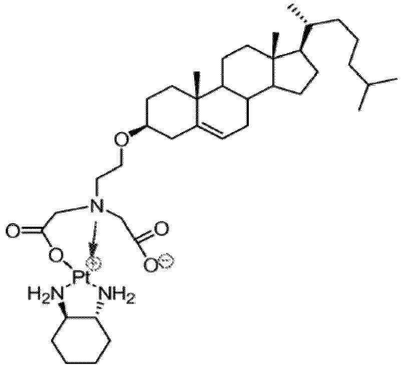

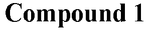

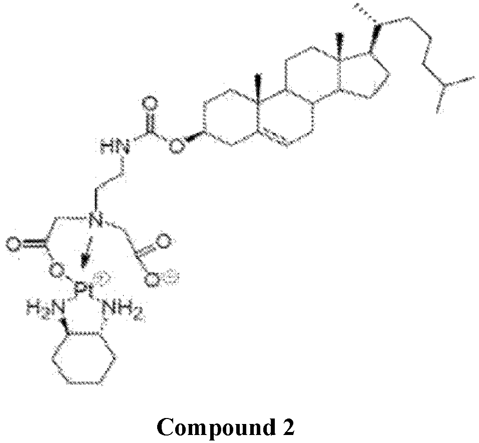

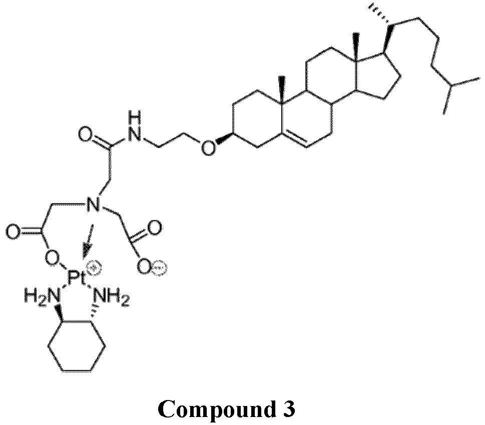

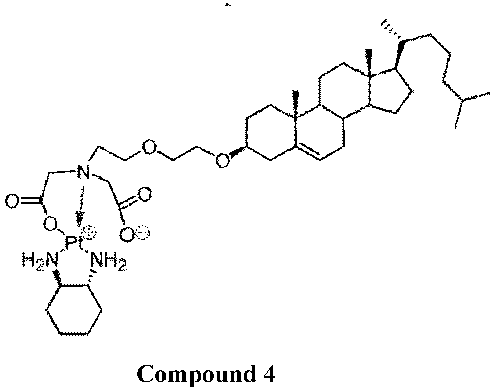

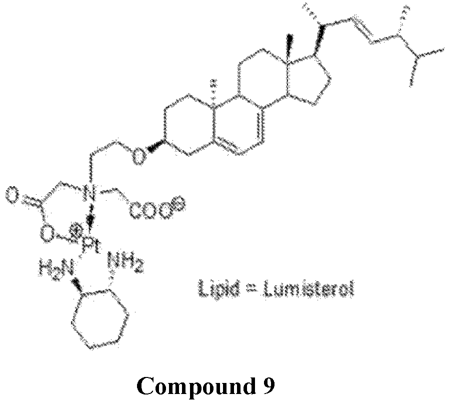

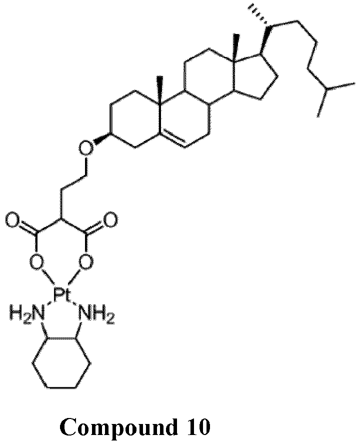

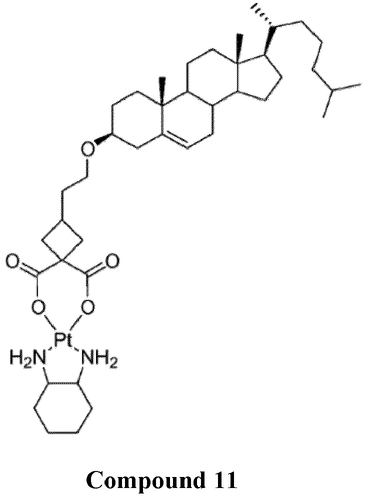

In another embodiment, the subject is a mammal, including human.

In another embodiment, the cancer is selected from a group consisting of breast cancer, ovarian cancer, glioma, gastrointestinal cancer, prostate cancer, carcinoma, lung carcinoma, hepatocellular carcinoma, testicular cancer, cervical cancer, endometrial cancer, bladder cancer, head and neck cancer, lung cancer, gastro-esophageal cancer and gynecological cancer, or any combination thereof.

In another embodiment, the compound of Formula I or Formula II is its derivative, salt form, tautomeric form, isomer, polymorph, solvate and intermediates thereof.

The lipid moiety in the compound of Formula I is a sterol, preferably a sterol selected from lumisterol, cholesterol, cholesterol chloroformate and derivatives thereof, or any combination thereof.

The lipid can be selected from sterol lipids. In some embodiments the lipid is lumisterol, cholesterol or cholesterol chloroformate.

In some embodiments, the lipid is cholesterol.

The linker in the compound of Formula I is -CH₂CH₂-, - CH₂CH₂NHC(O)-, - CH₂C(O)NHCH₂CH₂-, -CH₂CH₂OCH₂CH₂-, -C(O)CH₂-, or - CH₂CH₂NHC(O)CH₂-.

In another embodiment, the compound of Formula I or Formula II is administered at dosage where the platinum concentration ranges from about 50 mg/m² to about 500 mg/m².

In another embodiment, the compound of Formula I or Formula II is administered via intravenous administration, intra articular administration, pancreatic duodenal artery administration, intraperitoneal administration, hepatoportal administration, oral administration or intramuscular administration; optionally along with pharmaceutically acceptable excipient(s).

In another embodiment, the excipient(s) is selected from a group consisting of granulating agents, binding agents, lubricating agents, disintegrating agents, sweetening agents, glidants, anti-adherents, anti-static agents, surfactants, anti-oxidants, gums, coating agents, coloring agents, flavouring agents, coating agents, plasticizers, preservatives, suspending agents, emulsifying agents, plant cellulosic material and spheronization agents, or any combination thereof.

In addition to the platinum compounds disclosed herein, the particle can comprise co-lipids and/stabilizers. Additional lipids can be included in the particles for a variety of purposes, such as to prevent lipid oxidation, to stabilize the bilayer, to reduce aggregation during formation or to attach ligands onto the particle surface. Any of a number of additional lipids and/or other components can be present, including amphipathic, neutral, cationic, anionic lipids, and programmable fusion lipids. Such lipids and/or components can be used alone or in combination. One or more components of particle can comprise a ligand, e.g., a targeting ligand.

In some embodiments, the particle further comprises a phospholipid. Without limitations, the phospholipids can be of natural origin, such as egg yolk or soybean phospholipids, or synthetic or semisynthetic origin. The phospholipids can be partially purified or fractionated to comprise pure fractions or mixtures of phosphatidyl cholines, phosphatidyl cholines with defined acyl groups having 6 to 22 carbon atoms, phosphatidyl ethanolamines, phosphatidyl inositols, phosphatidic acids, phosphatidyl serines, sphingomyelin or phosphatidyl glycerols. Suitable phospholipids include, but are not limited to, phosphatidylcholine, phosphatidylglycerol, lecithin, β,γ-dipalmitoyl-α-lecithin, sphingomyelin, phosphatidylserine, phosphatidic acid, N-(2,3-di(9-(Z)-octadecenyloxy))-prop-1-yl-N,N,N-trimethylammonium chloride, phosphatidylethanolamine, lysolecithin, lysophosphatidylethanolamine, phosphatidylinositol, cephalin, cardiolipin, cerebrosides, dicetylphosphate, dioleoylphosphatidylcholine, dipalmitoylphosphatidylcholine, dipalmitoylphosphatidylglycerol, dioleoylphosphatidylglycerol, palmitoyl-oleoyl-phosphatidylcholine, di-stearoyl-phosphatidylcholine, stearoyl-palmitoyl-phosphatidylcholine, di-palmitoyl-phosphatidylethanolamine, di-stearoyl-phosphatidylethanolamine, di-myrstoyl-phosphatidylserine, di-oleyl-phosphatidylcholine, dimyristoyl phosphatidyl choline (DMPC), dioleoylphosphatidylethanolamine (DOPE), palmitoyloleoylphosphatidylcholine (POPC), egg phosphatidylcholine (EPC), distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), - phosphatidylethanolamine (POPE), dioleoyl-phosphatidylethanolamine 4-(N-maleimidomethyl)-cyclohexane-1-carboxylate (DOPE-mal), 1-stearoyl-2-oleoyl phosphatidylcholine (SOPC), 1,2-distearoyl-sn-glycem-3-phosphoethanolamine (DSPE), and any combinations thereof. Non-phosphorus containing lipids can also be used. These include, e.g., stearylamine, docecylamine, acetyl palmitate, fatty acid amides, and the like. Other phosphorus-lacking compounds, such as sphingolipids, glycosphingolipid families, diacylglycerols, and β-acyloxyacids, can also be used

In some embodiments, the phospholipid in the particle is selected from the group consisting of 1,2-Didecanoyl-sn-glycero-3-phosphocholine; 1,2-Dierucoyl-sn-glycero-3-phosphate (Sodium Salt); 1,2-Dierucoyl-sn-glycero-3-phosphocholine; 1,2-Dierucoyl-sn-glycero-3-phosphoethanolamine; 1,2-Dierucoyl-sn-glycero-3[Phospho-rac-(1-glycerol) (Sodium Salt); 1,2-Dilinoleoyl-sn-glycero-3-phosphocholine; 1,2-Dilauroyl-sn-glycero-3-phosphate (Sodium Salt); 1,2-Dilauroyl-sn-glycero-3-phosphocholine; 1,2-Dilauroyl-sn-glycero-3-phosphoethanolamine; 1,2-Dilauroyl-sn-glycero-3[Phospho-rac-(1-glycerol) (Sodium Salt); 1,2-Dilauroyl-sn-glycero-3[Phospho-rac-(1-glycerol) (Ammonium Salt); 1,2-Dilauroyl-sn-glycero-3-phosphoserine (Sodium Salt); 1,2-Dimyristoyl-sn-glycero-3-phosphate (Sodium Salt); 1,2-Dimyristoyl-sn-glycero-3-phosphocholine; 1,2-Dimyristoyl-sn-glycero-3-phosphoethanolamine; 1,2-Dimyristoyl-sn-glycero-3[Phospho-rac-(1-glycerol) (Sodium Salt); 1,2-Dimyristoyl-sn-glycero-3[Phospho-rac-(1-glycerol) (Ammonium Salt); 1,2-Dimyristoyl-sn-glycero-3[Phospho-rac-(1-glycerol) (Sodium/Ammonium Salt); 1,2-Dimyristoyl-sn-glycero-3-phosphoserine (Sodium Salt); 1,2-Dioleoyl-sn-glycero-3-phosphate (Sodium Salt); 1,2-Dioleoyl-sn-glycero-3-phosphocholine; 1,2-Dioleoyl-sn-glycero-3-phosphoethanolamine; 1,2-Dioleoyl-sn-glycero-3[Phospho-rac-(1-glycerol) (Sodium Salt); 1,2-Dioleoyl-sn-glycero-3-phosphoserine (Sodium Salt); 1,2-Dipalmitoyl-sn-glycero-3-phosphate (Sodium Salt); 1,2-Dipalmitoyl-sn-glycero-3 -phosphocholine; 1,2-Dipalmitoyl-sn-glycero-3-phosphoethanolamine; 1,2-Dipalmitoyl-sn-glycero-3[Phospho-rac-(1-glycerol) (Sodium Salt); 1,2-Dipalmitoyl-sn-glycero-3[Phospho-rac-(1-glycerol) (Ammonium Salt); 1,2-Dipalmitoyl-sn-glycero-3-phosphoserine (Sodium Salt); 1,2-Distearoyl-sn-glycero-3-phosphate (Sodium Salt); 1,2-Distearoyl-sn-glycero-3-phosphocholine; 1,2-Distearoyl-sn-glycero-3-phosphoethanolamine; 1,2-Distearoyl-sn-glycero-3 [Phospho-rac-(1-glycerol) (Sodium Salt); 1,2-Distearoyl-sn-glycero-3[Phospho-rac-(1-glycerol) (Ammonium Salt); 1,2-Distearoyl-sn-glycero-3-phosphoserine (Sodium Salt); Egg-PC; Hydrogenated Egg PC; Hydrogenated Soy PC; 1-Myristoyl-sn-glycero-3-phosphocholine; 1-Palmitoyl-sn-glycero-3-phosphocholine; 1-Stearoyl-sn-glycero-3-phosphocholine; 1-Myristoyl-2-palmitoyl-sn-glycero 3-phosphocholine; 1-Myristoyl-2-stearoyl-sn-glycero-3-phosphocholine; 1-Palmitoyl-2-myristoyl-sn-glycero-3-phosphocholine; 1-Palmitoyl-2-oleoyl-sn-glycero-3-phosphocholine; 1-Palmitoyl-2-oleoyl-sn-glycero-3-phosphoethanolamine; 1-Palmitoyl-2-oleoyl-sn-glycero-3[Phospho-rac-(1-glycerol)] (Sodium Salt); 1-Palmitoyl-2-stearoyl-sn-glycero-3-phosphocholine; 1-Stearoyl-2-myristoyl-sn-glycero-3-phosphocholine; 1-Stearoyl-2-oleoyl-sn-glycero-3-phosphocholine; and 1-Stearoyl-2-palmitoyl-sn-glycero-3-phosphocholine. In some embodiments, the phospholipid is SPOC, egg PC, or Hydrogenated Soy PC (HSPC). In one, the phospholipid in the composition is HSPC.

In some embodiments, the particle further comprises a polyethylene glycol (PEG). The PEG can be included in the particle by itself or conjugated with a component present in the particle. For example, the PEG can be conjugated with the platinum based compound or a co-lipid/stabilizer component of the particle. In some embodiments, the PEG is conjugated with a co-lipid component of the particle. Without limitations, the PEG can be conjugated with any co-lipid. For example, the PEG conjugated co-lipid can be selected from the group consisting of PEG conjugated diacylglycerols and dialkylglycerols, PEG- conjugated phosphatidylethanolamine, PEG conjugated to phosphatidic acid, PEG conjugated ceramides (see, U.S. Patent No. 5,885,613), PEG conjugated dialkylamines, PEG conjugated 1,2-diacyloxypropan-3-amines, and PEG conjugated to 1,2-distearoyl-sn-glycem-3-phosphoethanolamine (DSPE), and any combinations thereof. In some embodiments, the PEG conjugated lipid is 1,2-distearoyl-sn-glycem-3-phosphoethanolamine-N-[amino(polyethylene glycol)-2000] (DSPE-PEG2000).

In some embodiments, the particle further comprises a surfactant. Surfactants find wide application in formulations such as emulsions (including microemulsions) and liposomes. The most common way of classifying and ranking the properties of the many different types of surfactants, both natural and synthetic, is by the use of the hydrophile/lipophile balance (HLB). The nature of the hydrophilic group (also known as the "head") provides the most useful means for categorizing the different surfactants used in formulations (Rieger, in Pharmaceutical Dosage Forms, Marcel Dekker, Inc., New York, N.Y., 1988, p. 285).

If the surfactant molecule is not ionized, it is classified as a nonionic surfactant. Nonionic surfactants find wide application in pharmaceutical and cosmetic products and are usable over a wide range of pH values. In general, their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The use of surfactants in drug products, formulations and in emulsions has been reviewed (Rieger, in Pharmaceutical Dosage Forms, Marcel Dekker, Inc., New York, N.Y., 1988, p. 285).

In some embodiments, the particle can further comprise acationic lipid. Exemplary cationic lipids include, but are not limited to, N,N-dioleyl-N,N-dimethylammonium chloride (DODAC), N,N-distearyl-N,N-dimethylammonium bromide (DDAB), N-(1-(2,3-dioleoyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2,3-dioleyloxy)propylamine (DODMA), 1,2-DiLinoleyloxy-N,N-dimethylaminopropane (DLinDMA), 1,2-Dilinolenyloxy-N,N-dimethylaminopropane (DLenDMA), 1,2-Dilinoleylcarbamoyloxy-3-dimethylaminopropane (DLin-C-DAP), 1,2-Dilinoleyoxy-3-(dimethylamino)acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S-DMA), 1-Linoleoyl-2-linoleyloxy-3-dimethylaminopropane (DLin-2-DMAP), 1,2-Dilinoleyloxy-3-trimethylaminopropane chloride salt (DLin-TMA.C1), 1,2-Dilinoleoyl-3-trimethylaminopropane chloride salt (DLin-TAP.C1), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DMA), 1,2-Dilinolenyloxy-N,N-dimethylaminopropane (DLinDMA), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1' -(2-(4-(2-((2-(bis(2-hydroxydodecyl)amino)ethyl)(2-hydroxydodecyl)amino)ethyl)piperazin-1-yl)ethylazanediyl)didodecan-2-ol (Tech Gi), or a mixture thereof.

In some embodiments, the particle further comprises a non-cationic lipid. The non-cationic lipid can be an anionic lipid or a neutral lipid including, but not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoylphosphatidylethanolamine (POPE), dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1-trans PE, 1-stearoyl-2-oleoyl-phosphatidyethanolamine (SOPE), cholesterol, or a mixture thereof.

The conjugated lipids that inhibits aggregation of particles can also be included in the particles disclosed herein. Such lipids include, but are not limited to, a polyethyleneglycol (PEG)-lipid including, without limitation, a PEG-diacylglycerol (DAG), a PEG-dialkyloxypropyl (DAA), a PEG-phospholipid, a PEG-ceramide (Cer), or a mixture thereof. The PEG-DAA conjugate can be, for example, a PEG-dilauryloxypropyl (C12), a PEG-dimyristyloxypropyl (C14), a PEG-dipalmityloxypropyl (C16), or a PEG-distearyloxypropyl (C18). The conjugated lipid that prevents aggregation of particles can be from 0.01 mol % to about 20 mol % or about 2 mol % of the total lipid present in the particle.

In another embodiment, the compound of Formula I or Formula II is formulated into a dosage form selected from a group consisting of injectable, tablet, lyophilized powder and liposomal suspension, or any combination thereof.

In another embodiment, the compound of Formula I or Formula II enhances expression of immunoglobulin kappa C in tumor microenvironment of the cancer subject.

In another embodiment, the compound of Formula I or Formula II is used for inducing immune memory in a subject having cancer in the treatment of cancers which leads to sustained inhibition of tumor growth, limiting disease progression such as metastasis or relapse, by induction of immune response mediated through immunopotentiating molecule(s), which thereby activate cytokine(s), B-cell(s), T-cell(s), monocyte(s), macrophage(s), Natural Killer cell(s), dendritic cell(s) or a combination thereof.

In another embodiment, treatment of cancers with compounds of Formula I or Formula II leads to sustained inhibition of tumor growth, limiting disease progression including metastasis or the relapse of cancer. Compounds of Formula I or II prevents metastasis or the relapse by triggering humoral immune response through B cell(s); such as Plasmablast, Plasma cell, Lymphoplasmacytoid cell, Memory B cell, Follicular B cell, Marginal zone B cell, B-1 cell, B-2 cell and Regulatory B cell or any combination thereof.

In another embodiment, the T-cell(s) is selected from a group consisting of T helper cells, Cytotoxic T cells, memory T cells, suppressor T cells, Natural killer T cells, Mucosal associated invariant T cells and Gamma delta T cells, or any combination thereof.

In another embodiment, the immune response is activated via nucleic acid adduct formation, preferably via a double-stranded DNA adduct, single-stranded DNA adduct, double-stranded RNA adduct, or single-stranded RNA adduct.

The present invention also relates to a compound of Formula I or II, or a salt thereof, for use in a method of inducing immune memory in a subject having cancer, in treating or managing cancer and preventing metastasis or relapse of the cancer in a subject comprising administering to said subject a therapeutically effective amount of said compound of Formula I or Formula II, or a salt thereof. The compound 1 is the preferred compound employed from the group of compounds depicted or encompassed by compound of formula I.

The present invention relates to a compound of Formula I or II, or a salt thereof, for use in a method of enhancing immune response of a subject suffering from cancer, wherein said method comprises treating the cancer with a therapeutically effective amount of compound of Formula I or Formula II, preferably by Compound 1.

The present invention provides a compound of Formula I or II, or a salt thereof, for use in a method of inducing immune memory in a subject having cancer, wherein said method is for treating cancer and preventing metastasis or cancer relapse or cancer recurrence in a subject by administering platinum based compound(s), such as a compound of Formula I or Formula II, which is a platinate supramolecule.

The present method of treating a tumor or cancer is performed such that the therapeutic effect obtained from supramolecular therapy is greater than the therapeutic effect obtained standard of care with cytotoxic drug and known immunomodulator. The present invention provides a method of modifying mammalian immune reactions, including enhancing immunity in a mammal and inducing B-cell mediated immune memory. A tumor antigen or nucleic acid adduct (generated through cytotoxic effect of the platinate drug or compound of Formula I or Formula II) which modulates an immune response is one which produces any form of immune stimulation, including, but not limited to, induction of cytokines, B-cell activation, T-cell activation, monocyte activation, macrophage activation, Natural Killer cell activation, dendritic cell activation etc.

In the present invention, the compounds of Formula I or II, or a salt thereof, for use in a method of inducing immune memory in a subject having cancer are provided for cancer therapy, wherein said cancer therapy completely regressed tumor in a murine breast cancer bearing animals. These experimental animals did not develop tumors or show metastasis despite challenge with a subsequent injection of tumor cells. However, treatment of non-tumor bearing animals with Compound of Formula I or Formula II did not attribute to tumor rejection. This suggests the induction of immune memory only in tumor bearing mice treated with Compound of Formula I, wherein the administration of Compound of Formula I modifies the tumor cells to express and/or secrete immunopotentiating molecule(s). These molecules would activate T-cells and facilitate differentiation of B-cells to plasma and subsequently memory B-cells. Immunohistological and molecular profiling of immune markers have shown similar results in murine lung adenocarcinoma model treated with Compound of Formula I or Formula II, suggesting that this would be effective in a similar manner in all cancer models.

The present invention further provides compounds of Formula I or II for use in a method of inducing immune memory in a subject having cancer, wherein administration of a platinum based compound or platinate compound or compound of formula I or Formula II, its derivative, salt form, tautomeric form, isomer, polymorph, solvate, or intermediates thereof not only induce cytotoxic cell death, leading to tumor regression, but also develop an immune memory. The compound of Formula I or Formula II induces immune memory by focally modulating the tumor immune contexture. Treatment with this compound induces immune memory in the treated groups, as no tumor growth is observed upon re-implantation of cancer cells.

Thus, it is disclosed herein that Compound of Formula I or Formula II, or its derivative(s), salt(s), tautomeric form(s), isomer(s), polymorph(s), solvate(s), or intermediate(s) thereof, will modulate host immune system by altering the expression of immune markers and increasing tumor immunogenicity by facilitating tumor-infiltration of humoral immune cells.

Compound of Formula I and Formula II of the present invention has the general formula as below:

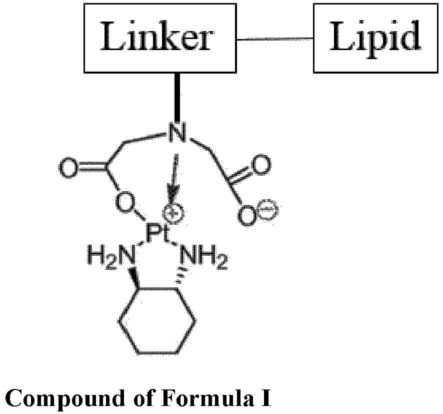

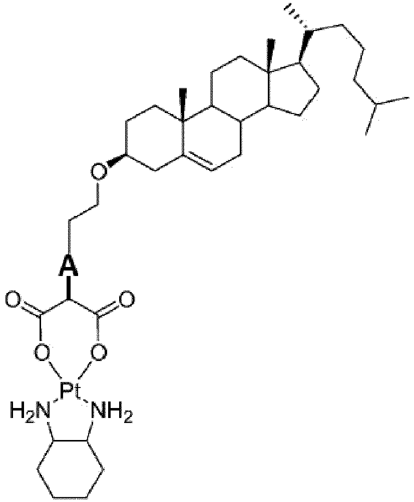

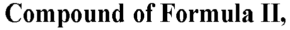

Wherein:
'A' is optionally present and wherein 'A' is cyclobutyl;
the lipid is a sterol; and
the linker is -CH₂CH₂-, -CH₂CH₂NHC(O)-, -CH₂C(O)NHCH₂CH₂-, - CH₂CH₂OCH₂CH_{2-,} -C(O)CH₂-, or -CH₂CH₂NHC(O)CH₂-..

The lipid moiety in the compound of Formula I or Formula II is a sterol, preferably a sterol selected from lumisterol, cholesterol, cholesterol chloroformate or derivatives thereof.

The linker in the compound of Formula I is selected from a group comprising -CH₂CH₂-, - CH₂CH₂NHC(O)-, -CH₂C(O)NHCH₂CH₂-, -CH₂CH₂OCH₂CH₂₋, -C(O)CH₂-, or -CH₂CH₂NHC(O)CH₂-.

Exemplary compounds of Formula (I) and Formula II include, but are not limited to the following compounds:

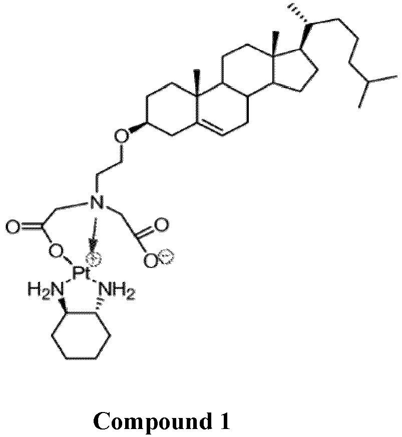

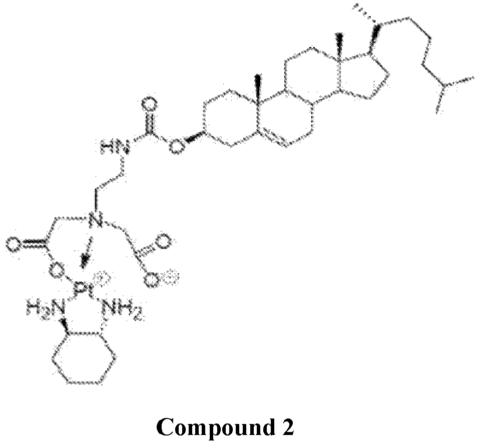

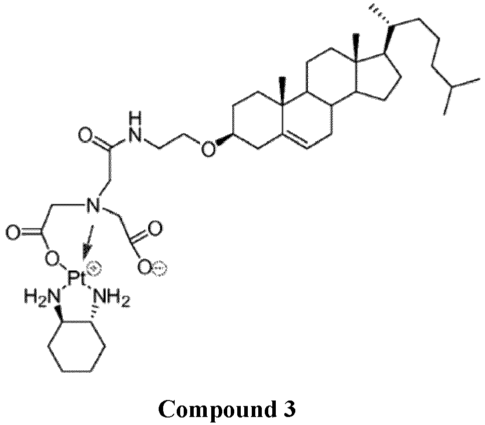

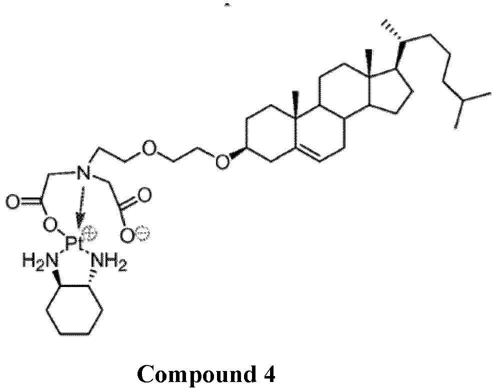

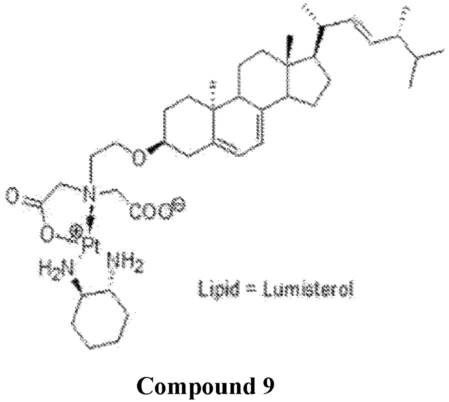

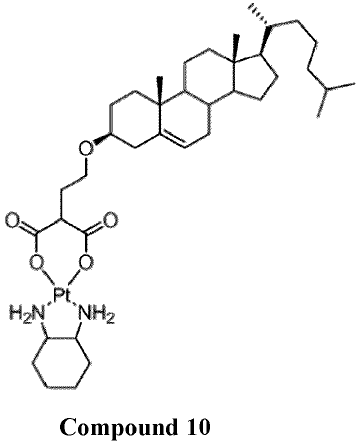

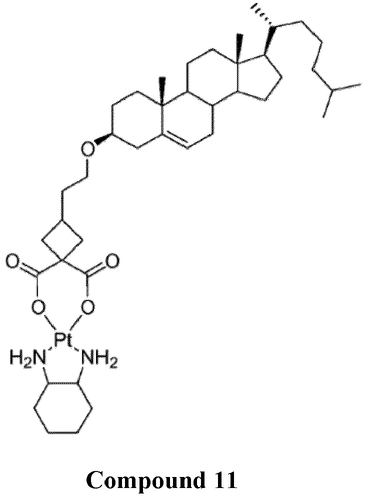

The Compound 1 of the Compound of Formula I is preferably employed in the present invention. Hereinafter, reference to Compound 1 *per se* implies that said compound is derived from the Compound of Formula I and used for experimentation purpose in the present invention.

Accordingly, in another aspect, described herein is a method of treating cancer or preventing cancer metastasis or relapse. Generally, the method comprises administering a therapeutically effective amount of a platinum based compounds, preferably Compound of Formula I or Formula II, more preferably a compound 1, disclosed herein to a subject in need thereof.

The phrase "therapeutically-effective amount" as used herein means that amount of a compound, material, or composition comprising a compound of the present invention which is effective for producing some desired therapeutic effect in at least a sub -population of cells in an animal at a reasonable benefit/risk ratio applicable to any medical treatment. Determination of a therapeutically effective amount is well within the capability of those skilled in the art. Generally, a therapeutically effective amount can vary with the subject's history, age, condition, sex, as well as the severity and type of the medical condition in the subject, and administration of other agents alleviate the disease or disorder to be treated.

Usually the amount of active compounds or the compound of formula I employed in the present invention, is between 0.1-95% by weight of the preparation, preferably between 0.2-20% by weight in preparations for parenteral use and preferably between 1 and 50% by weight in preparations for oral administration.

Toxicity and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compositions that exhibit large therapeutic indices are preferred. As used herein, the term ED denotes effective dose and is used in connection with animal models. The term EC denotes effective concentration and is used in connection with in vitro models.

The data obtained from the cell culture assays and animal studies can be used in formulating a range of dosage for use in humans. The dosage of such compounds lies preferably within a range of circulating concentrations that include the ED50 with little or no toxicity. The dosage can vary within this range depending upon the dosage form employed and the route of administration utilized.

The therapeutically effective dose can be estimated initially from cell culture assays. A dose can be formulated in animal models to achieve a circulating plasma concentration range that includes the IC50 (i.e., the concentration of the therapeutic which achieves a half-maximal inhibition of symptoms) as determined in cell culture. Levels in plasma can be measured, for example, by high performance liquid chromatography. The effects of any particular dosage can be monitored by a suitable bioassay.

The dosage can be determined by a physician and adjusted, as necessary, to suit observed effects of the treatment. Generally, the compositions are administered so that the agent/compound of Formula I is given at a dose where the platinum concentration is from about 50 mg/m² to about 500 mg/m². It is to be understood that ranges given here include all intermediate ranges, for example, the range 50 mg/m² to about 500 mg/m²includes 50 mg/m², 51 mg/m², 52 mg/m² and so on, until 500 mg/m². It is to be further understood that the ranges intermediate to the given above are also within the scope of this invention, for example, in the range 100 mg/m² to 110 mg/m² does range such as 101 mg/m² to 109 mg/m², and the like are included.

In some embodiments, the compositions are administered at a dosage so that the agent has an in vivo concentration of less than 200µM, less than 500nM, less than 400nM, less than 300 nM, less than 250 nM, less than 200 nM, less than 150 nM, less than 100 nM, less than 50 nM, less than 25 nM, less than 20, nM, less than 10 nM, less than 5nM, less than 1 nM, less than 0.5 nM, less than 0.lnM, less than 0.05nM, less than 0.01, nM, less than 0.005 nM, less than 0.001 nM after 15 mins, 30 mins, 1 hr, 1.5 hrs, 2 hrs, 2.5 hrs, 3 hrs, 4 hrs, 5 hrs, 6 hrs, 7 hrs, 8 hrs, 9 hrs, 10 hrs, 11 hrs, 12 hrs or more of time of administration.

With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment or make other alteration to treatment regimen. The dosing schedule can vary from once a week to daily depending on a number of clinical factors, such as the subject's sensitivity to the polypeptides. The desired dose can be administered everyday or every second, third, fourth, fifth, or sixth day. The desired dose can be administered at one time or divided into subdoses, e.g., 2-4 subdoses and administered over a period of time, e.g., at appropriate intervals through the day or other appropriate schedule. Such sub-doses can be administered as unit dosage forms. In some embodiments of the aspects described herein, administration is chronic, e.g., one or more doses daily over a period of weeks or months. Examples of dosing schedules are administration daily, twice daily, three times daily or four or more times daily over a period of 1 week, 2 weeks, 3 weeks, 4 weeks, 1 month, 2 months, 3 months, 4 months, 5 months, or 6 months or more.

As used herein, the term "administer" refers to the placement of a composition into a subject by a method or route which results in at least partial localization of the composition at a desired site such that desired effect is produced. A compound or composition described herein can be administered by any appropriate route known in the art including, but not limited to, oral or parenteral routes, including intravenous, intramuscular, subcutaneous, transdermal, airway (aerosol), pulmonary, nasal, rectal, and topical (including buccal and sublingual) administration.

Exemplary modes of administration include, but are not limited to, injection, infusion, instillation, inhalation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrastemal injection and infusion. In some embodiments, the compositions are administered by intravenous infusion, oral mode or via injection.

As used herein, the term "cancer" refers to an uncontrolled growth of cells that may interfere with the normal functioning of the bodily organs and systems. Cancers that migrate from their original location and seed vital organs can eventually lead to the death of the subject through the functional deterioration of the affected organs. Metastasis is a cancer cell or group of cancer cells, distinct from the primary tumor location resulting from the dissemination of cancer cells from the primary tumor to other parts of the body. At the time of diagnosis of the primary tumor mass, the subject may be monitored for the presence of in transit metastases, e.g., cancer cells in the process of dissemination. As used herein, the term cancer, includes, but is not limited to the following types of cancer, breast cancer, biliary tract cancer, bladder cancer, brain cancer including Glioblastomas and medulloblastomas; cervical cancer; choriocarcinoma; colon cancer; endometrial cancer; esophageal cancer, gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma, Wilms tumor. Examples of cancer include but are not limited to, carcinoma, including adenocarcinoma, lymphoma, blastoma, melanoma, sarcoma, and leukemia. More particular examples of such cancers include squamous cell cancer, small-cell lung cancer, non-small cell lung cancer, gastrointestinal cancer, Hodgkin's and non-Hodgkin's lymphoma, pancreatic cancer, Glioblastoma, cervical cancer, ovarian cancer, liver cancer such as hepatic carcinoma and hepatoma, bladder cancer, breast cancer, colon cancer, colorectal cancer, endometrial carcinoma, salivary gland carcinoma, kidney cancer such as renal cell carcinoma and Wilms' tumors, basal cell carcinoma, melanoma, prostate cancer, vulval cancer, thyroid cancer, testicular cancer, esophageal cancer, and various types of head and neck cancer. Other cancers will be known to the artisan.

As used herein, the term "cancer" includes, but is not limited to, solid tumors and blood born tumors. The term cancer refers to disease of skin, tissues, organs, bone, cartilage, blood and vessels. The term "cancer" further encompasses primary and metastatic cancers. Examples of cancers that can be treated with the compounds of the invention include, but are not limited to, carcinoma, including that of the bladder, breast, colon, kidney, lung, ovary, pancreas, stomach, cervix, thyroid, and skin, including squamous cell carcinoma; hematopoietic tumors of lymphoid lineage, including, but not limited to, leukemia, acute lymphocytic leukemia, acute lymphoblastic leukemia, B-cell lymphoma, T-cell lymphoma, Hodgkins lymphoma, non-Hodgkins lymphoma, hairy cell lymphoma, and Burketts lymphoma; hematopoietic tumors of myeloid lineage including, but not limited to, acute and chronic myelogenous leukemias and promyelocytic leukemia; tumors of mesenchymal origin including, but not limited to, fibrosarcoma, rhabdomyosarcoma, and osteosarcoma; other tumors including melanoma, seminoma, tetratocarcinoma, neuroblastoma, and glioma; tumors of the central and peripheral nervous system including, but not limited to, astrocytoma, neuroblastoma, glioma, and schwannomas; and other tumors including, but not limited to, xenoderma, pigmentosum, keratoactanthoma, thyroid follicular cancer, and teratocarcinoma. The methods disclosed herein are useful for treating patients who have been previously treated for cancer, as well as those who have not previously been treated for cancer. Indeed, the methods and compositions of this invention can be used in first-line and second- line cancer treatments.

The methods described herein relate to treating a subject having or diagnosed as having cancer. Subjects having cancer can be identified by a physician using current methods of diagnosing cancer. Symptoms and/or complications of cancer which characterize these conditions and aid in diagnosis are well known in the art and include but are not limited to, growth of a tumor, impaired function of the organ or tissue harboring cancer cells, etc. Tests that may aid in a diagnosis of, e.g. cancer include, but are not limited to, tissue biopsies and histological examination. A family history of cancer, or exposure to risk factors for cancer (e.g. tobacco products, radiation, etc.) can also aid in determining if a subject is likely to have cancer or in making a diagnosis of cancer.

For administration to a subject, the platinum based compounds and/or particles comprising said platinum based compounds are provided in pharmaceutically acceptable compositions. Accordingly, the disclosure also provides pharmaceutical compositions comprising the platinum based compounds or particles as disclosed herein. These pharmaceutically acceptable compositions comprise a therapeutically-effective amount of one or more of the platinum based compounds or particles described herein, formulated together with one or more pharmaceutically acceptable carriers (additives) and/or diluents. The said pharmaceutical compositions of the present invention are specially formulated for administration in solid or liquid form, including those adapted for the following: (1) oral administration, for example, drenches (aqueous or non-aqueous solutions or suspensions), lozenges, dragees, capsules, pills, tablets (e.g., those targeted for buccal, sublingual, and systemic absorption), boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) transmucosally; or (9) nasally. Additionally, the compounds of the present disclosure can be implanted into a patient or injected using a drug delivery system.

In some embodiments, the pharmaceutical composition comprising a platinum based compound can be a parenteral dose form. Since administration of parenteral dosage forms typically bypasses the patient's natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions. In addition, controlled-release parenteral dosage forms can be prepared for administration of a patient, including, but not limited to, DUROS^{®}-type dosage forms and dose-dumping.

Suitable vehicles that can be used to provide parenteral dosage forms of a composition as described herein are well known to those skilled in the art. Examples include, without limitation: sterile water; water for injection USP; saline solution; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate.

Compounds that alter or modify the solubility of a pharmaceutically acceptable salt can also be incorporated into the parenteral dosage forms of the disclosure, including conventional and controlled-release parenteral dosage forms.

Pharmaceutical compositions can also be formulated to be suitable for oral administration, for example as discrete dosage forms, such as, but not limited to, tablets (including without limitation scored or coated tablets), pills, caplets, capsules, chewable tablets, powder packets, cachets, troches, wafers, aerosol sprays, or liquids, such as but not limited to, syrups, elixirs, solutions or suspensions in an aqueous liquid, a non-aqueous liquid, an oil-in-water emulsion, or a water-in-oil emulsion. Such compositions contain a predetermined amount of the pharmaceutically acceptable salt of the disclosed compounds, and may be prepared by methods of pharmacy well known to those skilled in the art. See generally, Remington: The Science and Practice of Pharmacy, 21st Ed., Lippincott, Williams, and Wilkins, Philadelphia PA. (2005).

Conventional dosage forms generally provide rapid or immediate drug release from the formulation. Depending on the pharmacology and pharmacokinetics of the drug, use of conventional dosage forms can lead to wide fluctuations in the concentrations of the drug in a patient's blood and other tissues. These fluctuations can impact a number of parameters, such as dose frequency, onset of action, duration of efficacy, maintenance of therapeutic blood levels, toxicity, side effects, and the like. Advantageously, controlled-release formulations can be used to control a drug's onset of action, duration of action, plasma levels within the therapeutic window, and peak blood levels. In particular, controlled- or extended-release dosage forms or formulations can be used to ensure that the maximum effectiveness of a drug is achieved while minimizing potential adverse effects and safety concerns, which can occur both from under-dosing a drug (i.e., going below the minimum therapeutic levels) as well as exceeding the toxicity level for the drug. In some embodiments, a composition as described herein can be administered in a sustained release formulation.

Other than in the operating examples, or where otherwise indicated, all numbers expressing quantities of ingredients or reaction conditions used herein should be understood as modified in all instances by the term "about." The term "about" when used in connection with percentages may mean ±5% of the value being referred to. For example, about 100 means from 95 to 105.

The terms "decrease", "reduced", "reduction", "decrease" or "inhibit" are all used herein generally to mean a decrease by a statistically significant amount. However, for avoidance of doubt, ""reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%>, or at least about 80%>, or at least about 90%> or up to and including a 100% decrease (e.g. absent level as compared to a reference sample), or any decrease between 10-100% as compared to a reference level.

The terms "increased" 'increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%>, or at least about 40%>, or at least about 50%>, or at least about 60%>, or at least about 70%), or at least about 80%>, or at least about 90%> or up to and including a 100%) increase or any increase between 10-100% as compared to a reference level, or at least about a 2-fold, or at least about a 3 -fold, or at least about a 4-fold, or at least about a 5 -fold or at least about a 10-fold increase, or any increase between 2-fold and 10-fold or greater as compared to a reference level.

As used herein, the terms "treat," "treatment," "treating," or "amelioration" refer to therapeutic treatments, wherein the object is to reverse, alleviate, ameliorate, inhibit, slow down or stop the progression or severity of a condition associated with a disease or disorder, e.g. cancer. The term "treating" includes reducing or alleviating at least one adverse effect or symptom of a condition, disease or disorder associated with a cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of, or at least slowing of, progress or worsening of symptoms compared to what would be expected in the absence of treatment. Beneficial or desired clinical results include, but are not limited to, alleviation of one or more symptom(s), diminishment of extent of disease, stabilized (i.e., not worsening) state of disease, delay or slowing of disease progression, amelioration or palliation of the disease state, remission (whether partial or total), and/or decreased mortality, whether detectable or undetectable. The term "treatment" of a disease also includes providing relief from the symptoms or side-effects of the disease (including palliative treatment).

As used herein, "management" or "managing" refers to preventing a disease or disorder from occurring in a subject, decreasing the risk of death due to a disease or disorder, delaying the onset of a disease or disorder, inhibiting the progression of a disease or disorder, partial or complete cure of a disease or disorder and/or adverse effect attributable to the said disease or disorder, obtaining a desired pharmacologic and/or physiologic effect (the effect may be prophylactic in terms of completely or partially preventing a disorder or disease or condition, or a symptom thereof and/or may be therapeutic in terms of a partial or complete cure for a disease or disorder and/or adverse effect attributable to the disease or disorder), relieving a disease or disorder (i.e. causing regression of the disease or disorder). Further, the present disclosure also envisages treating the said disease by administering the therapeutic composition of the instant disclosure.

The terms "subject" and "individual" are used interchangeably herein, and mean a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. Patient or subject includes any subset of the foregoing, e.g., all of the above, but excluding one or more groups or species such as humans, primates or rodents. In certain embodiments, the subject is a mammal, e.g., a primate, e.g., a human. The terms, "patient" and "subject" are used interchangeably herein. The terms, "patient" and "subject" are used interchangeably herein.

Preferably, the subject is a mammal. The mammal can be a human, non-human primate, mouse, rat, dog, cat, horse, or cow, but are not limited to these examples. Mammals other than humans can be advantageously used as subjects that represent animal models of cancer. In addition, the methods described herein can be used to treat domesticated animals and/or pets. A subject can be male or female. A subject can be one who has been previously diagnosed with or identified as suffering from cancer, but need not have already undergone treatment.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

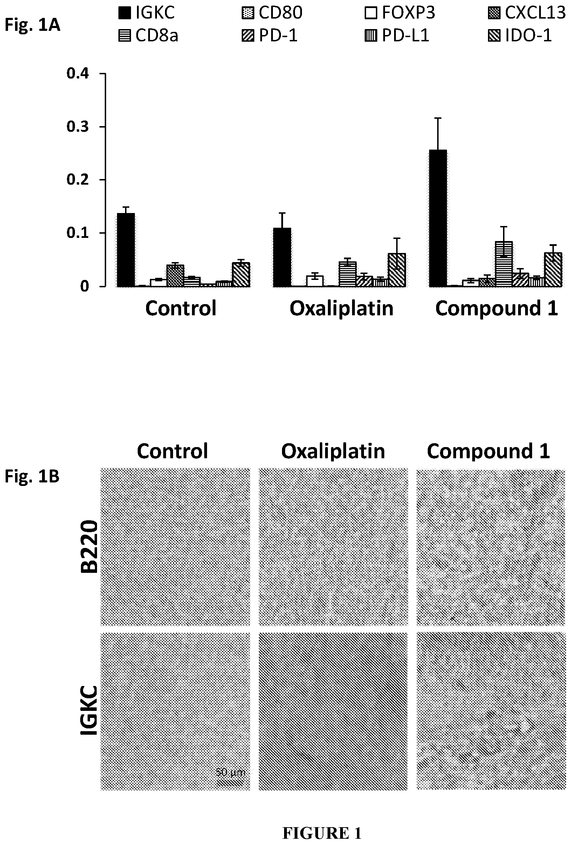

Portions of harvested tumors from different treatment groups were used for total RNA isolation and subsequently followed by qRT-PCR, the results of which indicate significant increase in immunoglobulin kappa C (IGKC) mRNA levels in tumors treated with Compound 1 (Fig. 1A). A prognostic impact of immunoglobulin kappa C (IGKC) Portions of harvested tumors from different treatment groups were used for total RNA isolation, followed by qRT-PCR, the results of which indicate significant increase in immunoglobulin kappa C (IGKC) mRNA levels in tumors treated with Compound 1 (Fig. 1A). A prognostic impact of IGKC expression has been described in cancer, where it has been shown to be a prognostic marker in human solid tumors (Schmidt et al., Clin Cancer Res 2012;18:2695-704; Whiteside and Ferrone, Clin Cancer Res. 2012 May 1;18(9):2417-9). These studies have established and support the emerging treatment concepts that exploit the humoral immune response (Lohr et al., Cancer Letters 333 (2013) 222-228). Immunohistochemical analysis of tumor sections from murine NSCLC tumor model, treated with Compound 1 show elevated levels of IGKC and B220 (Fig. 1B), suggesting recruitment of humoral immune cells.

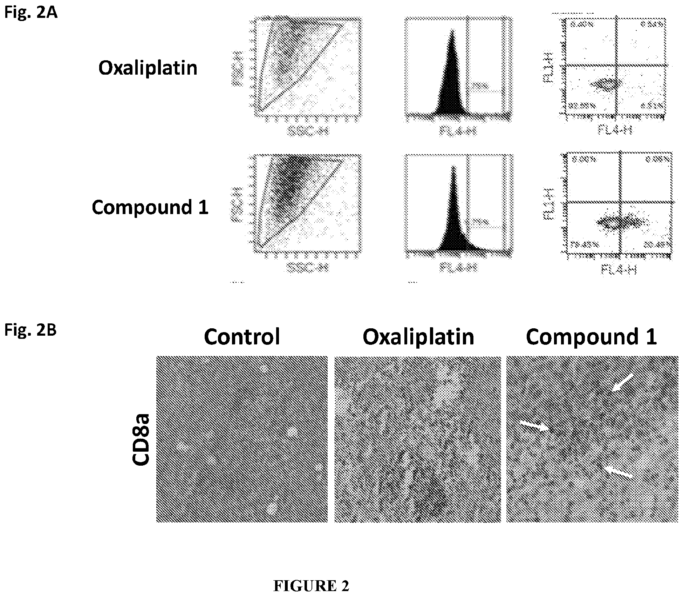

Many clinical trials of cancer immunotherapies have shown tumor shrinking and prolonged survival. However, to keep cancer away for the long term, the immune system should remember how to recognize and attack the cancer cells, if they come back in future. Hence, an "immunological memory" would empower the body's fight against recurrence of cancer. Immune memory cells are poised to rapidly expand and induce effector functions upon recurrence, while existing in a functionally quiescent state. The paradigm is that memory T cells remain inactive due to lack of T cell receptor (TCR) stimuli, where regulatory T cells (Treg) often orchestrate memory T cell quiescence (Kalia et al., Immunity 42, 1116-1129, June 16, 2015). Loss of Treg cells in addition to activation of effector T cells and memory CD8+ T cells would generate protective efficacy. Treatment with Compound 1, induces substantial activation of TCR, in comparison to Oxaliplatin (Fig. 2A), with infiltration of cytotoxic T cells, established through detection of CD8+ T cells (Fig. 2B)

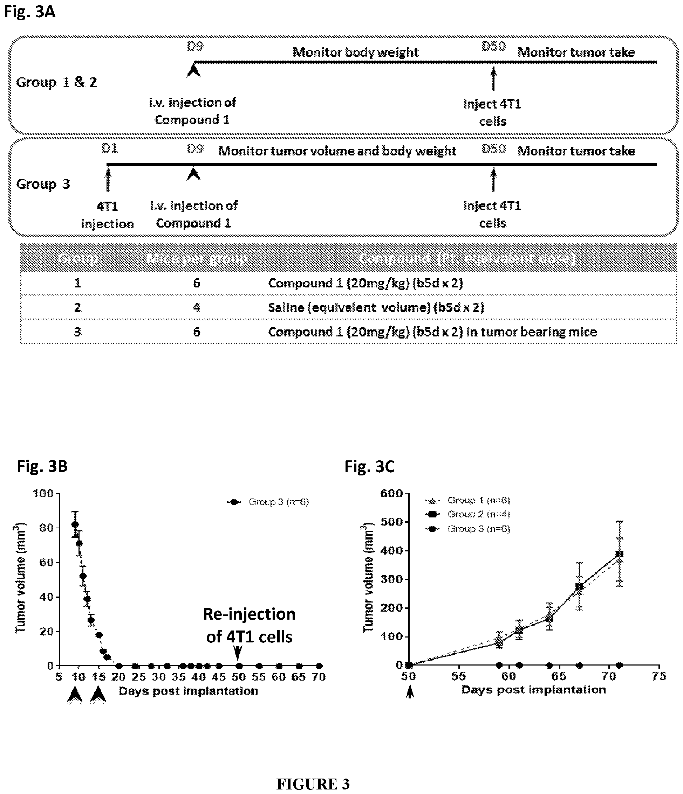

These events should induce "immunological memory" and prevent recurrence of cancer. In order to check this hypothesis, immune memory in Compound 1 treated tumors were examined. The study plan is schematically shown in Fig. 3A. Results indicate that treatment with Compound 1 induces immune memory in the treated animals, as no tumor growth was observed upon re-implantation of cancer cells into animals, which had previously undergone tumor regression with Compound 1 treatment (Fig. 3B). The results also ruled out the effect of any residual drug inducing tumor memory in non-tumor bearing animals.

The mechanism of action of platinum drugs is primarily via coordination to DNA forming adducts, disrupting DNA replication and transcription, subsequently leading to cell death through apoptosis (Fink et al., Cancer Res, 1997, 57: 1841-1845; Takahara et al., J. Am. Chem. Soc. 1996, 118, 12309; Silverman et al., J. Biol. Chem. 2002, 277, 49743). The binding of platinum drugs to DNA and oligonucleotides have been characterized in detail (Reedijk, Proc. Natl. Acad. Sci. U.S.A. 2003, 100, 3611; Reedijk, Curr. Opin. Chem. Biol. 1999, 3, 236; Guo and Sadler, J. AdV. Inorg. Chem. 2000, 49, 183). The adduct formation by platinum drugs have often raised the question whether this is specific to DNA or can they also form RNA adducts. This query was supplemented by the observation that fluorescently labelled cisplatin was also detected in nucleolus, in addition to lysosome, Golgi and secretory compartments in the cell (Safaei et al., Clin Cancer Res. 2005 Jan 15;11(2 Pt 1):756-67). A subsequent study concluded that cisplatin treatment could generate platinum adducts in the internal loop and other unusual cross-links in structurally complex RNAs and was stable for a long period to induce changes in RNA-dependent biological processes. (Hostetter et al., J. Am. Chem. Soc., 2009, 131 (26), pp 9250-9257). In a study in yeast, it was established that platinum accumulates on RNA, including poly(A)-mRNA, rRNA, forming adducts (Hostetter et al., ACS Chem. Biol., 2012, 7 (1), pp 218-225). The platinum accumulation in cellular RNA is greater than in DNA. These observations significantly add a new repertoire to the cellular effects of platinum drugs, as disruption of RNA and small molecule-RNA interactions could disrupt processes regulated by RNA (Chapman et al., J. Am. Chem. Soc., 2010, 132 (6), pp 1946-1952). Small double stranded RNAs (dsRNAs) have also been shown to activate immune pathways in mammalian cells (Gantier and Williams, Cytokine Growth Factor Rev. 2007; 18(5-6): 363-371; Chiappinelli et al., Cell. 2015 Aug 27;162(5):974-86).

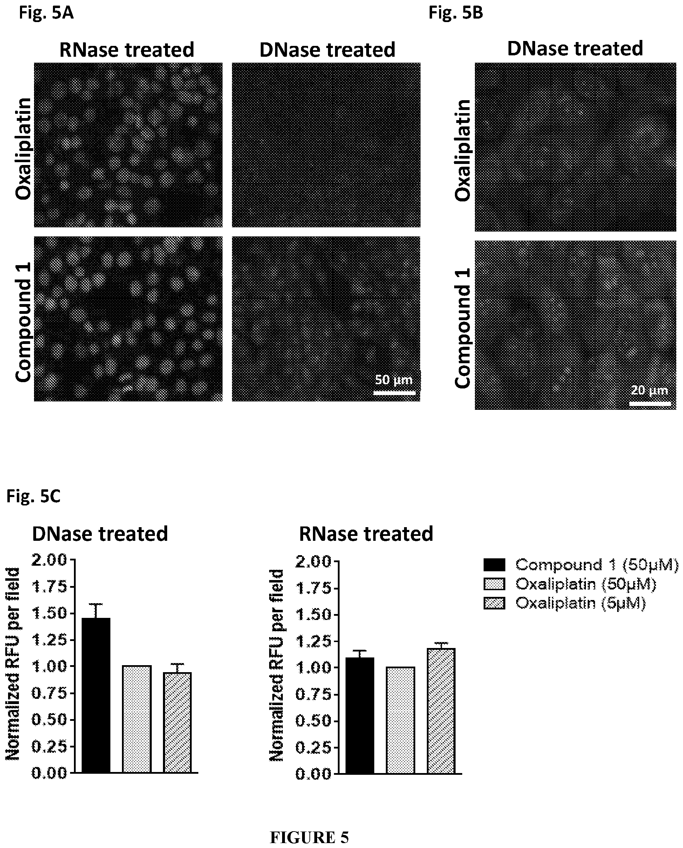

Upon treatment of cancer cells with Compound 1 and Oxaliplatin followed by treatment with DNase or RNase, the cells were stained with propidium iodide that would intercalate only in double stranded nucleic acid, hence stain only dsRNA (Fig. 5A and 5B).

The DNase treated samples showed an enhancement of relative fluorescent intensity per field indicating an elevated amount dsRNA in Compound 1 treated cells (Fig. 5C). Compared to Oxaliplatin, Compound 1 induced more stable RNA adducts that could potentially induce an immune response by activation of the TLR pathway, as small dsRNAs have been shown to activate these immune pathways in mammalian cells (Gantier and Williams, Cytokine Growth Factor Rev. 2007; 18(5-6): 363-371; Chiappinelli et al., Cell. 2015 Aug 27;162(5):974-86).

Taken together, studies of the present invention show that supramolecular therapeutics, especially Compound 1 or its derivative, salt, tautomeric form, isomer, polymorph, solvate, or intermediates thereof, can emerge as a unique approach to focally modulate the tumor immune contexture in a subject. Compounds 2-11 of the present invention are also indicated to provide similar immune memory response as provided by the Compound 1, when employed in the experiments.

### Advantages of the method of the present invention:

While the immuno-oncology space is focused on adaptive and innate immunity, our findings show that compound of Formula I and Formula II have emerged as the first of its class to mount a humoral response, creating tremendous value in the clinic. It exhibits a T-cell mediated immune response similar to standard of care. However, this effect was less pronounced than the recruitment of humoral immune cells into the tumor, which can potentially prevent relapse. Indeed, TNBC patients with higher IGKC have been associated with long term survival. Moreover, no tumor growth was observed upon re-implantation of cancer cells into treatment groups that had previously undergone tumor regression, demonstrating that compound of Formula I can trigger immune memory.

The above information further exemplified by non-limiting examples below demonstrate that Compound of Formula I and/or Formula II, preferably Compound 1 induces immune memory through a unique humoral immune response alongside a T-cell mediated effect. The following examples, are only illustrative in nature and should not be construed to limit the scope of the present invention in any manner.

### EXAMPLES

### Example 1

### This example demonstrates that treatment of Compound of Formula I (Compound 1) induces

### B-cell mediated immune response in tumors.

4T1 cells were subcutaneously implanted in Balb/c mice to generate tumors. When tumors reached an average volume of 100 mm³, they were treated with either Compound 1 or Oxaliplatin. After one cycle of treatment, following regression, tumors were harvested and a portion of the tumor from each group was used for total RNA isolation. The tumor infiltrating immune cells were evaluated for relative mRNA expression levels of immune activating and immune suppressive genes (Denkert et al., Clin Oncol. 2015; 33(9):983-91).

Results indicate significant increase in IGKC mRNA levels in tumors treated with Compound 1 (Fig. 1A). A prognostic impact of IGKC expression has been described in cancer, where it has been shown to be a prognostic marker in human solid tumors (Schmidt et al., Clin Cancer Res 2012;18:2695-704; Whiteside and Ferrone, Clin Cancer Res. 2012 May 1;18(9):2417-9).

Murine NSCLC tumor model was generated by subcutaneously implanting LLC cells in C57BL6 mice. Treatment with either Compound 1 or Oxaliplatin was initiated when average tumor volume reached 100 mm³. Following two cycles of treatment, tumors were harvested and a portion fixed in formalin. FFPE sections were generated from the fixed tumors and immunohistochemical analysis of tumor sections was carried out for both T and B-cells.

Treatment with Compound 1 shows elevated levels of IGKC and B220 (Fig. 1B), suggesting recruitment of humoral immune cells. Studies have established and supported the emerging treatment concepts that exploit the humoral immune response (Lohr et al., Cancer Letters 333 (2013) 222-228) and current results corroborate those observations.

### Example 2

### This example demonstrates that treatment of Compound of Formula I (Compound 1) induces T-cell mediated immune response in tumors.

Many clinical trials of cancer immunotherapies have shown tumor shrinkage and prolonged survival. The paradigm is that memory T cells remain inactive due to lack of T cell receptor (TCR) stimuli, where regulatory T (Treg) cells often orchestrate memory T cell quiescence (Kalia et al., Immunity 42, 1116-1129, June 16, 2015). Loss of Treg cells in addition to activation of effector T cells and memory CD8+ T cells would generate protective efficacy.

Treatment with Compound 1 induces substantial activation of TCR, in comparison to Oxaliplatin (Fig. 2A), with infiltration of cytotoxic T cells, established through detection of CD8+ T cells (Fig. 2B)

### Example 3

### This example demonstrates that treatment of Compound of Formula I (Compound 1) induces immunological memory.

To keep cancer away for the long term, the immune system should remember how to recognize and attack the cancer cells, if they come back in future. Hence, an "immunological memory" would empower the body's fight against recurrence of cancer.

4T1 cells were subcutaneously implanted in Balb/c mice to generate tumors. When tumors reached an average volume of 100 mm³, they were treated with Compound 1. Two groups of Balb/c mice (non-tumor bearing) were either treated with Compound 1 or saline (designated Group 1 and 2 respectively; Fig. 3A). The detailed study plan has been schematically shown in Fig. 3A.

Immune memory cells are poised to rapidly expand and induce effector functions upon recurrence, while existing in a functionally quiescent state. In order to check this hypothesis, we examined immune memory in Compound 1 treated tumors. Results indicate that treatment with Compound 1 induces immune memory in the treated animals, as no tumor growth was observed upon re-implantation of cancer cells into animals (Group 3), which had previously undergone tumor regression with Compound 1 treatment (Fig. 3B). Non-tumor bearing Balb/c mice, treated with Compound 1, when re-implanted with 4T1 cells led to the growth of tumors (Group 1), similar to those observed for saline treated mice (Group 2) (Fig. 3C). The results also rule out the effect of any residual drug inducing tumor memory in non-tumor bearing animals.

To understand the role of immune components in tumor regression following treatment with Compound 1, tumor regression was studied in three different mice strains. These included immunocompetent mice (Balb/c); B cell-deficient mice (designated Jh^{-/-}) and mice lacking functional B cells and T cells (SCID). 4T1 cells were subcutaneously implanted in the three strains mentioned and when tumors reached an average volume of 100 mm³, they were divided into two groups. One group was kept as control and the other treated with Compound 1 and the tumor volume in all the animals were recorded.

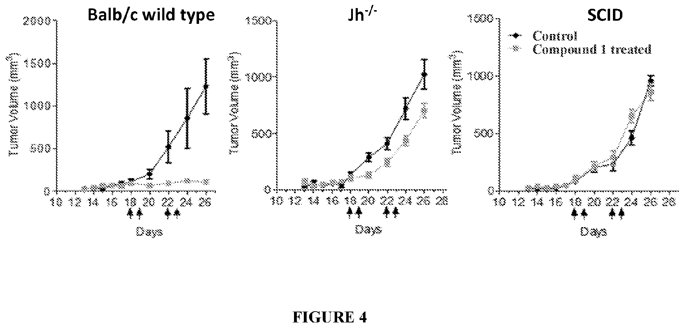

Results indicate that tumor Compound 1 causes tumor regression only in immunocompetent mice (Fig. 4). Tumors do not regress in mice lacking immune cells and their growth is similar to the control mice.

Taken together, studies in the present invention show that supramolecular therapeutics, especially Compound 1 or its derivative, salt, tautomeric form, isomer, polymorph, solvate, or intermediates thereof, can emerge as a novel approach to focally modulate the tumor immune contexture.

### Example 4

### This example demonstrates that treatment with Compound of Formula I (Compound 1) generates ds-RNA adducts.

The mechanism of action of platinum drugs is primarily via coordination to DNA forming adducts, disrupting DNA replication and transcription, subsequently leading to cell death through apoptosis (Fink et al., Cancer Res, 1997, 57: 1841-1845; Takahara et al., J. Am. Chem. Soc. 1996, 118, 12309; Silverman et al., J. Biol. Chem. 2002, 277, 49743). The binding of platinum drugs to DNA and oligonucleotides have been characterized in detail (Reedijk, Proc. Natl. Acad. Sci. U.S.A. 2003, 100, 3611; Reedijk, Curr. Opin. Chem. Biol. 1999, 3, 236; Guo and Sadler, J. AdV. Inorg. Chem. 2000, 49, 183). The adduct formation by platinum drugs have often raised the question whether this is specific to DNA or can they also form RNA adducts. This query was supplemented by the observation that fluorescently labelled cisplatin was also detected in nucleolus, in addition to lysosome, Golgi and secretory compartments in the cell (Safaei et al., Clin Cancer Res. 2005 Jan 15;11(2 Pt 1):756-67). A subsequent study concluded that cisplatin treatment could generate platinum adducts in the internal loop and other unusual cross-links in structurally complex RNAs and was stable for a long period to induce changes in RNA-dependent biological processes. (Hostetter et al., J. Am. Chem. Soc., 2009, 131 (26), pp 9250-9257). In a study in yeast, it was established that platinum accumulates on RNA, including poly(A)-mRNA, rRNA, forming adducts (Hostetter et al., ACS Chem. Biol., 2012, 7 (1), pp 218-225). The platinum accumulation in cellular RNA is greater than in DNA. These observations significantly add a new repertoire to the cellular effects of platinum drugs, as disruption of RNA and small molecule-RNA interactions could disrupt processes regulated by RNA (Chapman et al., J. Am. Chem. Soc., 2010, 132 (6), pp 1946-1952). Small dsRNAs have also been shown to activate immune pathways in mammalian cells (Gantier and Williams, Cytokine Growth Factor Rev. 2007; 18(5-6): 363-371; Chiappinelli et al., Cell. 2015 Aug 27;162(5):974-86).

4T1 cells were grown on a coverslip in RPMI media to 70-80% confluency. The cells were treated with Compound 1 or Oxaliplatin. Detection of ds-RNA was a modification of protocol described by Kantarjian et al. (Kantarjian et al., Blood. 1985 Jul;66(1):39-46). Following treatment, the coverslips were washed in phosphate-buffered saline (PBS) and fixed in 70% ice-cold ethanol. Cells were then washed with saline and treated with 1 mg/mL of DNase in 0.25 mol/L sucrose, 5 mmol/L MgC1₂, and 20 mmol/L Tris-HCL (pH 6.5) at 37°C for 60 min. In addition, another set of cells, subjected to Compound 1 or Oxaliplatin treatment were treated with RNase at a concentration of 5 mg/mL at 37°C for 60 minutes. After another wash in PBS, the coverslips were exposed to propidium iodide at a final concentration of 50µg/ml, diluted in a solution containing 10 mmol/L Tris-HCI (pH 7.4) and 5 mmol/L MgCl₂. Stained cells were kept at 4°C prior to microscopic analysis using a NIKON epi-fluorescence microscope using excitation wavelengths of 480 nm and emission wavelengths of 590 nm.

Cells, treated with DNase and stained with propidium iodide would intercalate only in double stranded nucleic acid, hence stain only dsRNA (Fig. 5A,B). The DNase treated samples showed an enhancement of relative fluorescent intensity per field indicating an elevated amount dsRNA in Compound 1 treated cells (Fig. 5C). Compared to Oxaliplatin, Compound 1 induced more stable RNA adducts that could potentially induce an immune response by activation of the TLR pathway, as small dsRNAs have been shown to activate these immune pathways in mammalian cells (Gantier and Williams, Cytokine Growth Factor Rev. 2007; 18(5-6): 363-371; Chiappinelli et al., Cell. 2015 Aug 27;162(5):974-86).

### Example 5

### This example demonstrates that treatment with Compound of Formula I (Compound 1) leads to B cell differentiation and activates TLRs in splenic B cells in tumor bearing mice.

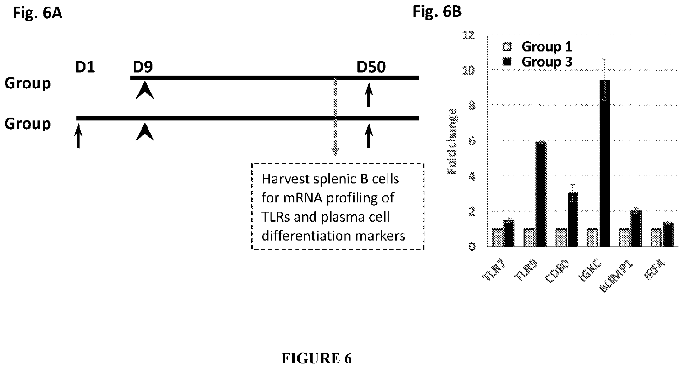

Mice splenic B cells were isolated from Group 1 and Group 3 mice, described in Example 3. A schematic representation of experimental detail in shown in Fig. 6A. Spleen was harvested from mice (n=3) and minced into small pieces in RPMI-1640 basal media. The pieces were placed on top of a 40 mesh membrane and crushed with the back of a syringe and the single cell suspension was collected onto a 50 ml tube. The single cell suspension was washed twice with PBS to remove debris by centrifuging at 2000 rpm. Splenocytes were counted using haemocytometer and 100 million splenocytes were resuspended in 1 ml of isolation buffer (2% FBS, 100 mM EDTA in DPBS) and transferred to a 5 ml polysterene tube. The B cells were isolated from splenocytes using the EasySep Stem Cell B cell isolation kit as per manufacturers protocol. Ten million B cells were used for RNA isolation, followed by relative mRNA expression levels of genes involved in B cell differentiation and TLR activation were evaluated.

Results indicate significant increase in IGKC mRNA levels in splenic B cells isolated from tumor bearing mice treated with Compound 1 (Fig. 6B). An elevated expression of TLRs and CD80 was also noted in these mice. Studies have suggested that both B-cell intrinsic and extrinsic TLRs can regulate B-cell responses *in vivo*, with role in B cell activation and differentiation, although the extent varies from one model system to another (Pasare et al. 2005, Nature. 2005;438:364-8; Hou et al. 2008, Immunity;29:272-82; Ruprecht et al. 2006, Eur J Immunol. 2006;36:810-6). Exposure of B cells to TLR ligands alone may be sufficient to promote numerous responses, including expression of activation markers such as CD69, CD80 and CD86, antigen presentation, proliferation, class switch recombination and antibody secretion (Jiang et al. 2007, Eur J Immunol;37:2205-13; Capolunghi et al. 2008, J Immunol;180:800-8; He et al., 2004, J Immunol;173:4479-91)

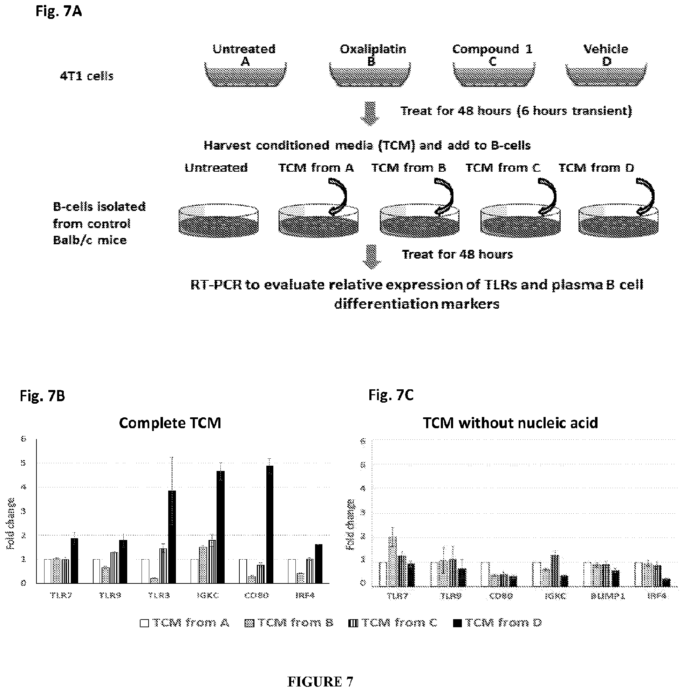

Naive human B cells express low levels of TLRs, whereas activated and memory B cells express significantly higher levels of TLRs (Agrawal and Gupta, 2010, J Clin Immunol;31:89-98; Bernasconi et al. 2003, Blood;101:4500-4). The expression pattern of TLRs in B cell subsets have a distinct pattern of expression, though the levels vary between individual subsets. The expression of TLRs in B cells is regulated by the action of cytokines and signalling from the BCRs. In addition, small fragments of nucleic acids or DNA and RNA adducts, serving as ligands for TLRs are also immunostimulatory towards B cells. To understand the factors/agents responsible for activating B-cell intrinsic TLRs, a study was designed as shown in Fig. 7A.

4T1 cells were seeded in cell culture dishes in RPMI-1640 media containing 10% FBS. The cells were treated with Oxaliplatin and Compound 1 when they reached 60 % confluence. Following a transient treatment of 6 hours, the media was removed and supplemented with fresh culture media. Cells were incubated at 37°C for 24 hours. After 24 hours, the treatment conditioned media (TCM) was collected from each treatment and divided into two parts. One part was passed through 0.1 micron PES filters to obtain tumor conditioned media free of cell debris, with nucleic acids intact; while the other was passed through 0.1 micron Nylon membrane filters, where the nucleic acids would stick and get removed. The filtration process through Nylon membrane was repeated twice to remove all nucleic acids from the TCM. The harvested TCMs were used immediately for conditioning of B cells, isolated from naive mice splenocytes using the EasySep Stem Cell B cell isolation kit as per manufacturers protocol. The B cells were incubated with conditioned media and harvested after 24 hours of incubation. RNA was isolated from the B cells and used for relative mRNA expression level evaluation of genes involved in B cell differentiation and TLR activation.

Results indicate that B cell differentiation markers and TLR activation markers were substantially increased in splenic B cells cultured with TCM from Compound 1 treated 4T1 cells (Fig. 7B). Interestingly, the relative level of markers was substantially reduced when nucleic acids were removed from TCM (Fig. 7C). This suggests that small fragments of nucleic acids or DNA and RNA adducts could serve as ligands for TLRs, which get activated and are immunostimulatory towards B cells. The role of B-cell intrinsic TLRs in regulating B-cell responses *in vivo* has already been demonstrated (Pasare et al. 2005, Nature. 2005;438:364-8; Ruprecht et al. 2006, Eur J Immunol. 2006;36:810-6). Studies have shown activation of TLR3 in response to ds-RNA (Alexopoulou et al., Nature; 2001; 413, 732-738). We have observed generation of ds-RNA adducts (Fig. 5), which could play a role in activation of TLR 3. However, the role of cytokines and other nucleic acid components have to be evaluated in this activation.

The above data demonstrates that Compound of Formula I, preferably Compound 1 induces immune memory through a unique humoral immune response alongside a T-cell mediated effect.

It is also to be understood that the phrases or terms employed herein are for the purpose of description and not intended to be of any limitation. Throughout the present invention, the word "comprise", or variations such as "comprises" or "comprising" wherever used, are to be understood to imply the inclusion of a stated element, integer or step, or group of elements, integers or steps, but not the exclusion of any other element, integer or step, or group of elements, integers or steps.

Where a numerical limit or range is stated herein, the endpoints are included. Also, values and sub-ranges within a numerical limit or range are specifically included as if explicitly written out.

With respect to the use of any plural and/or singular terms in the present invention, those of skill in the art can translate from the plural to the singular and/or from the singular to the plural as is considered appropriate to the context and/or application. The various singular/plural permutations may be expressly set forth herein for the sake of clarity.

Any discussion of documents, articles and the like that has been included in this specification is solely for the purpose of providing a context for the present invention. It is not to be taken as an admission that any or all of these matters form a part of the prior art base or are common general knowledge in the field relevant to the present invention, as it existed anywhere before the priority date of this application.

## Claims

1. A compound of Formula I or II, or a salt thereof, for use in a method of inducing immune memory in a subject having cancer, comprising administering an amount of a compound of Formula I or II, or a salt thereof, to the subject: 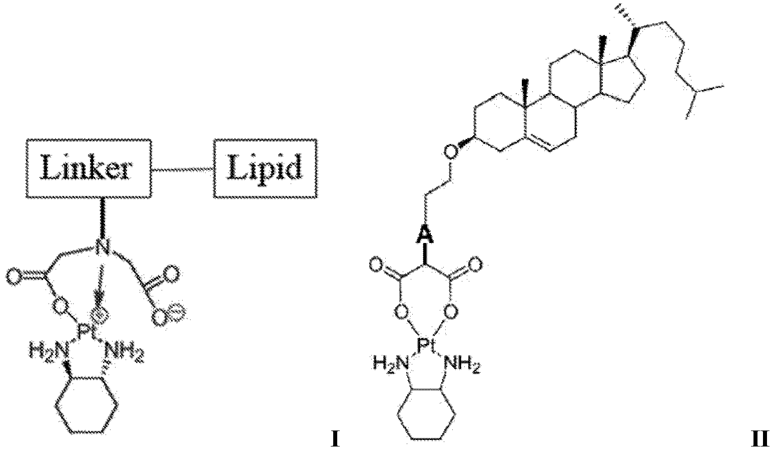 wherein:
A is absent or cyclobutyl;
the lipid is a sterol; and
the linker is -CH₂CH₂-, -CH₂CH₂NHC(O)-, -CH₂C(O)NHCH₂CH₂-, - CH₂CH₂OCH₂CH₂-, -C(O)CH₂-, or -CH₂CH₂NHC(O)CH₂-.

2. A compound for use according to claim 1, wherein the compound induces B-cell mediated immune memory in the subject.

3. A compound for use according to any one of the preceding claims, wherein the compound is of formula (I):

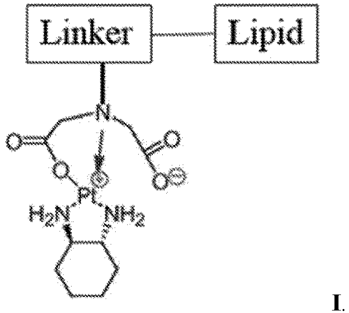

4. A compound for use according to any one of the preceding claims, wherein the lipid is lumisterol, cholesterol or cholesterol chloroformate.

5. A compound for use according to claim 1 or claim 2, wherein the compound is of formula (II):

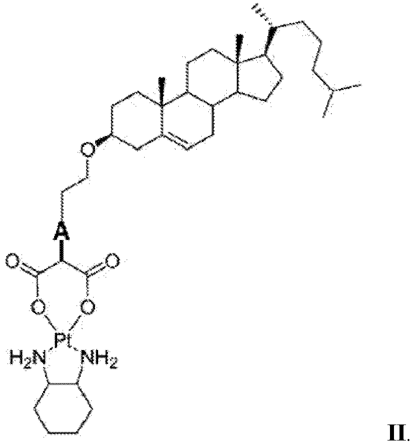

6. A compound for use according to claim 1 or claim 2, wherein the compound is: 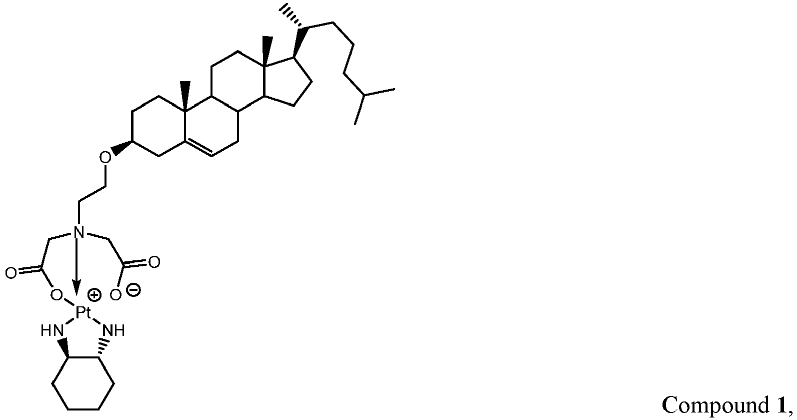 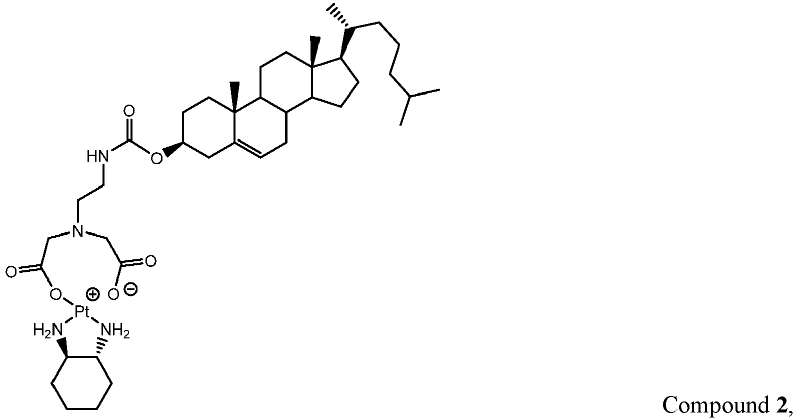 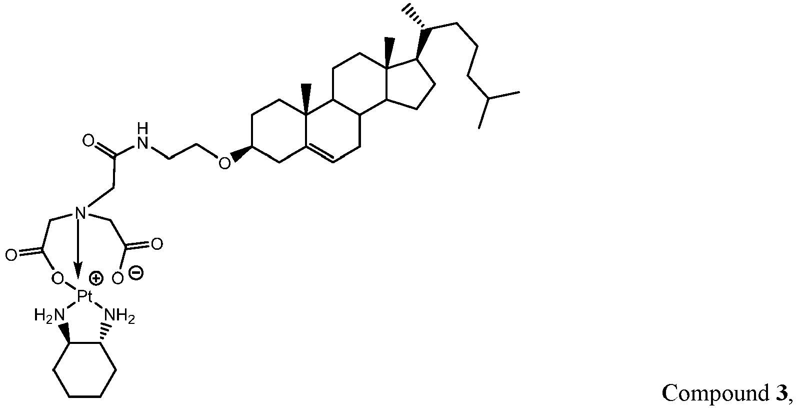 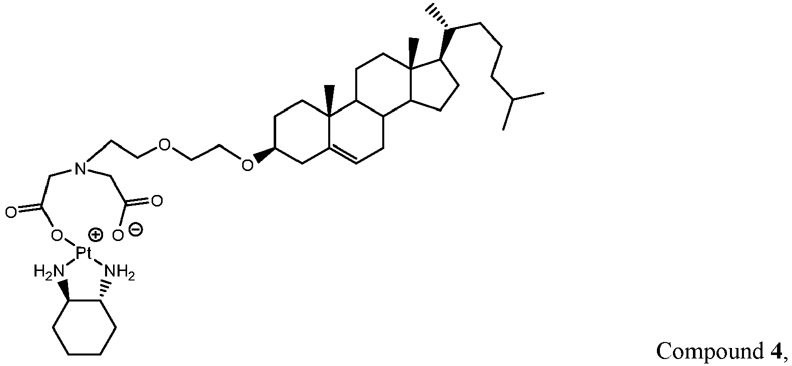 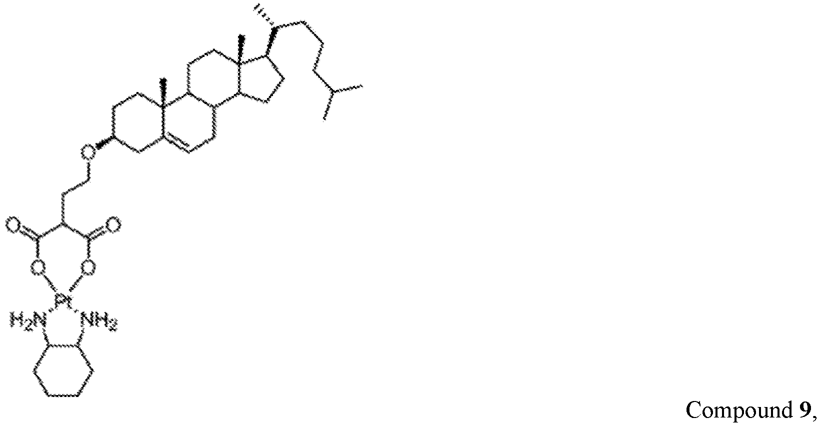 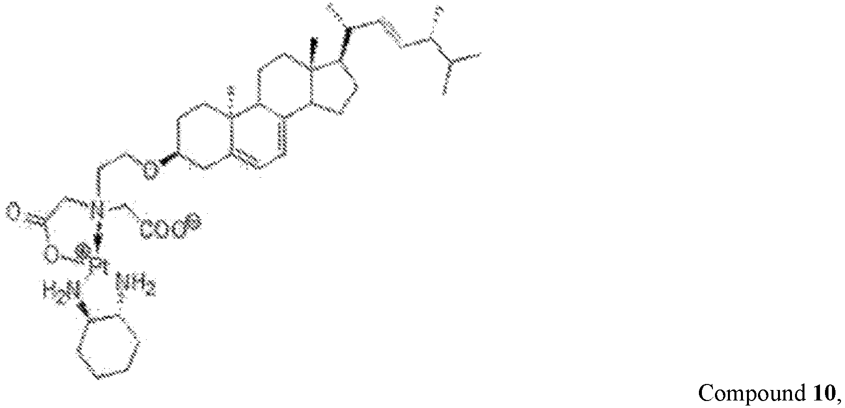 or 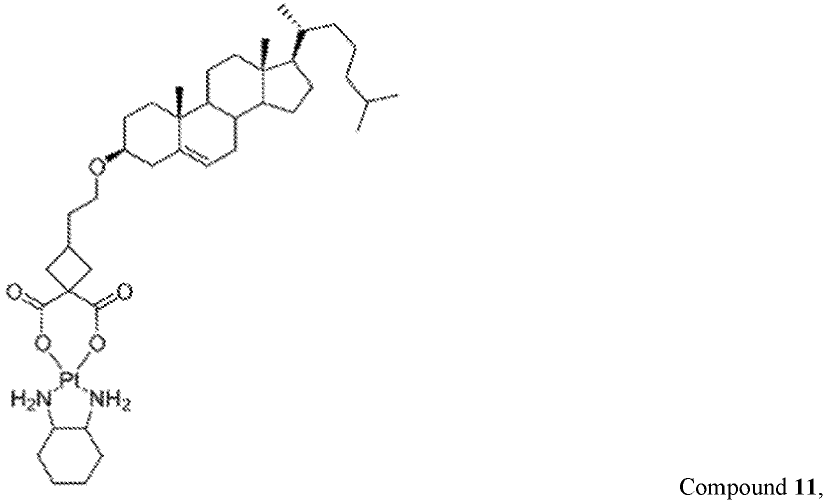 or a salt thereof.

7. A compound for use according to claim 1 or 2, wherein the compound is Compound 1 or a salt thereof:

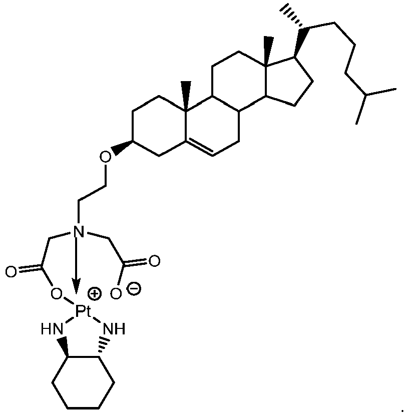

8. A compound for use according to any one of claims 1 or 3 to 7, wherein the cancer is breast cancer, ovarian cancer, glioma, gastrointestinal cancer, prostate cancer, carcinoma, lung carcinoma, hepatocellular carcinoma, testicular cancer, cervical cancer, endometrial cancer, bladder cancer, head and neck cancer, lung cancer, gastro-esophageal cancer, gynecological cancer, or a combination thereof.

9. A compound for use according to any one of the preceding claims, wherein the amount of the compound provides a platinum concentration of about 50 mg/m² to about 500 mg/m².

10. A compound for use according to any one of the preceding claims, wherein the compound is administered intravenously, intraarticularly, pancreatic duodenal arterally, intraperitoneally, hepatoportally, orally, or intramuscularly.

11. A compound for use according to any one of the preceding claims, wherein the compound is formulated in a dosage form that is an injection, tablet, lyophilized powder, liposomal suspension, or a combination thereof.

12. A compound for use according to any one of the preceding claims, wherein the administration enhances expression of immunoglobulin kappa C.

13. A compound for use to any one of the preceding claims, wherein induction of the immune memory is mediated through one or more immunopotentiating molecules, optionally wherein the immunopotentiating molecule activates cytokines, B-cells, T-cells, monocytes, macrophages, natural killer cells, dendritic cells, or a combination thereof.

14. A compound for use according to claim 13, wherein the administration triggers a humoral response through B-cells, optionally wherein the B-cells are plasmablasts, plasma cells, lymphoplasmacytoid cells, memory B-cells, follicular B-cells, marginal zone B-cells, B-1 cells, B-2 cells, regulatory B-cells, or a combination thereof; or wherein the T-cells are T helper cells, cytotoxic T cells, memory T cells, suppressor T cells, natural killer T cells, mucosal associated invariant T cells, gamma delta T cells, or a combination thereof.

15. A compound of use according to any one of the preceding claims, wherein induction of the immune memory is activated by the formation of a nucleic acid adduct, optionally wherein the nucleic acid adduct is a double-stranded DNA adduct, single-stranded DNA adduct, double-stranded RNA adduct, or a single-stranded RNA adduct.

## Patentansprüche

1. Verbindung der Formel I oder II oder ein Salz davon zur Verwendung in einem Verfahren zur Anregung des Immungedächtnisses in einem Individuum, das die Verabreichung einer Menge einer Verbindung der Formel I oder II: 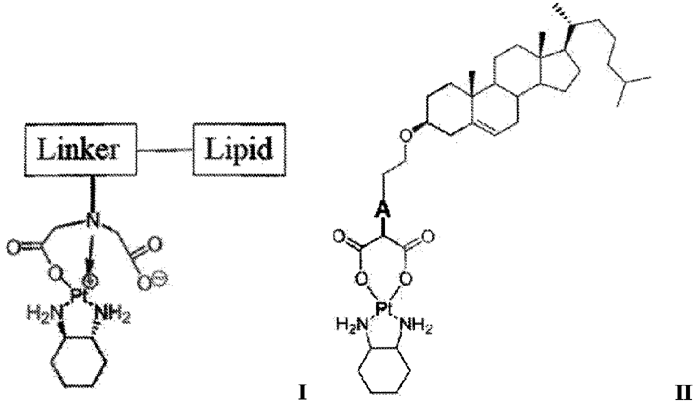 oder eines Salzes davon an das Individuum umfasst, wobei:
A fehlt oder Cyclobutyl ist;
das Lipid ein Sterin ist und
der Linker -CH₂CH₂-, -CH₂CH₂NHC(O)-, -CH₂C(O)NHCH₂CH₂-, - CH₂CH₂OCH₂CH₂-, -C(O)CH₂- oder -CH₂CH₂NHC(O)CH₂- ist.

2. Verbindung zur Verwendung nach Anspruch 1, wobei die Verbindung im Individuum ein B-Zell-vermitteltes Immungedächtnis induziert.

3. Verbindung zur Verwendung nach einem der vorhergehenden Ansprüche, wobei die Verbindung Formel (I) aufweist:

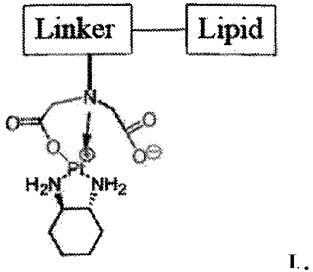

4. Verbindung zur Verwendung nach einem der vorhergehenden Ansprüche, wobei das Lipid Lumisterol, Cholesterin oder Cholesterinchlorformiat ist.

5. Verbindung zur Verwendung nach Anspruch 1 oder Anspruch 2, wobei die Verbindung die Formel (II) aufweist:

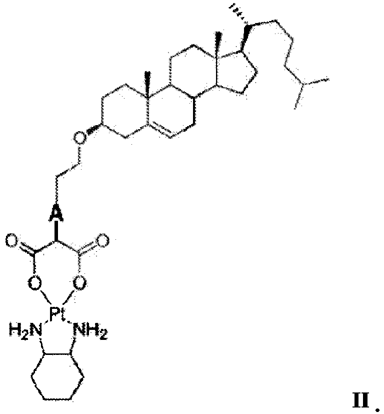

6. Verbindung zur Verwendung nach Anspruch 1 oder Anspruch 2, wobei die Verbindung: 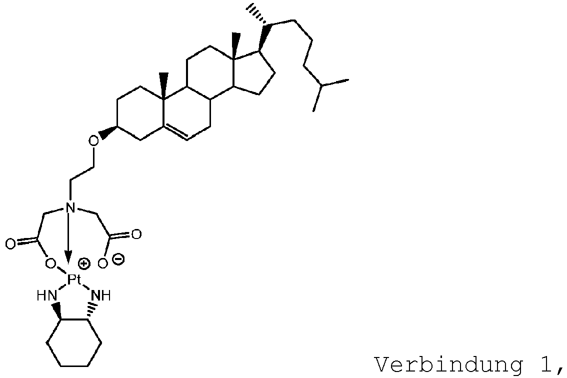 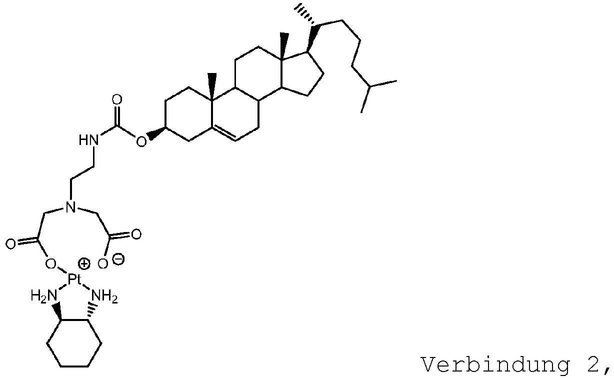 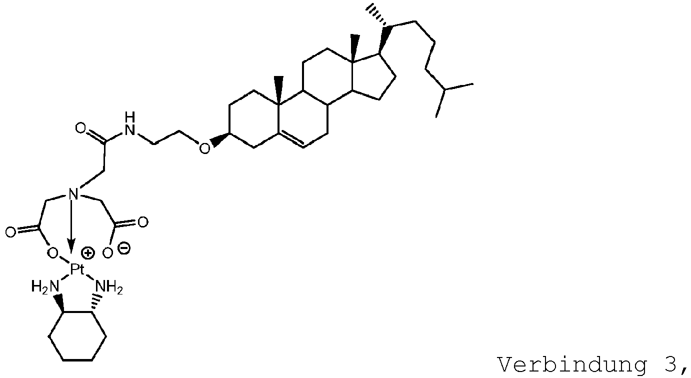 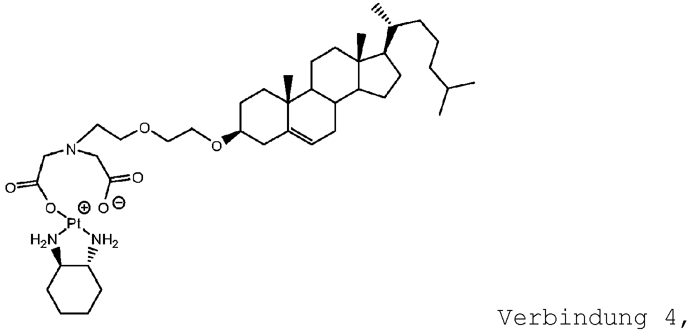 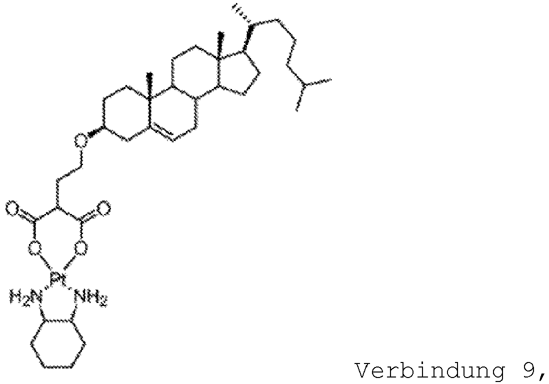 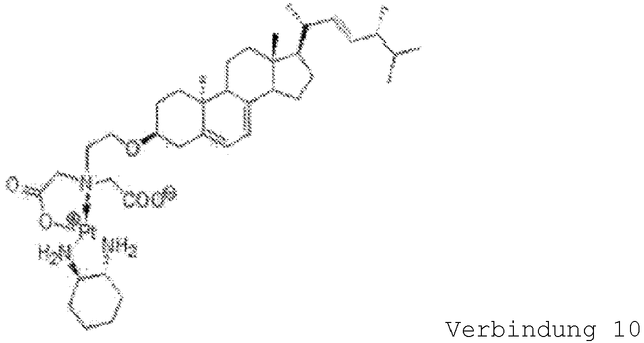 oder 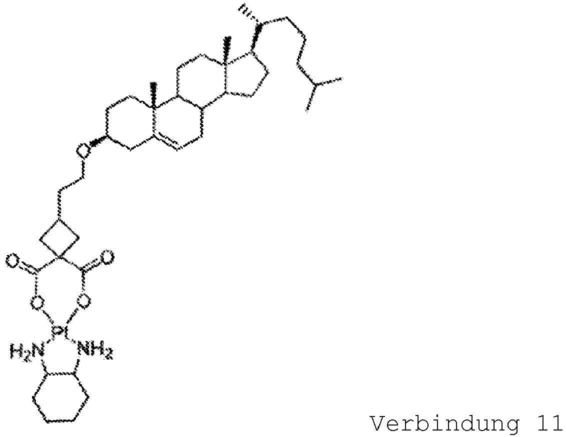 oder ein Salz davon ist.

7. Verbindung zur Verwendung nach Anspruch 1 oder 2, wobei die Verbindung Verbindung 1 oder ein Salz davon ist:

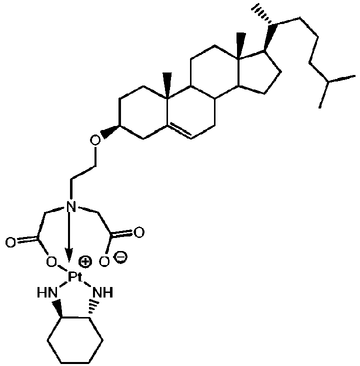

8. Verbindung zur Verwendung nach einem der Ansprüche 1 oder 3 bis 7, wobei der Krebs Brustkrebs, Ovarialkrebs, ein Gliom, gastrointestinaler Krebs, Prostatakrebs, ein Karzinom, Lungenkarzinom, hepatozelluläres Karzinom, Hodenkrebs, ein Zervixkarzinom, endometriales Karzinom, Blasenkrebs, Kopf- und Halskrebs, Lungenkrebs, ein gastroösophageales Karzinom, gynäkologischer Krebs oder eine Kombination davon ist.

9. Verbindung zur Verwendung nach einem der vorhergehenden Ansprüche, wobei die Menge der Verbindung eine Platinkonzentration von etwa 50 mg/m² bis etwa 500 mg/m² ergibt.

10. Verbindung zur Verwendung nach einem der vorhergehenden Ansprüche, wobei die Verbindung intravenös, intraartikulär, über eine Arteria pancreaticoduodenalis, intraperitoneal, hepatoportal, oral oder intramuskulär verabreicht wird.

11. Verbindung zur Verwendung nach einem der vorhergehenden Ansprüche, wobei die Verbindung in einer Dosierungsform formuliert wird, bei der es sich um eine Injektion, Tablette, ein lyophilisiertes Pulver, eine liposomale Suspension oder eine Kombination davon handelt.

12. Verbindung zur Verwendung nach einem der vorhergehenden Ansprüche, wobei die Verabreichung die Expression von Immunglobulin Kappa C verstärkt.

13. Verbindung zur Verwendung nach einem der vorhergehenden Ansprüche, wobei eine Anregung des Immungedächtnisses durch ein oder mehrere immunpotenzierende Moleküle vermittelt wird, wobei das immunpotenzierende Molekül gegebenenfalls Zytokine, B-Zellen, T-Zellen, Monozyten, Makrophagen, natürliche Killerzellen, dendritische Zellen oder eine Kombination davon aktiviert.

14. Verbindung zur Verwendung nach Anspruch 13, wobei die Verabreichung eine humorale Antwort über B-Zellen auslöst, wobei es sich bei den B-Zellen gegebenenfalls um Plasmablasten, Plasmazellen, lymphoplasmozytoide Zellen, Gedächtnis-B-Zellen, follikuläre B-Zellen, Marginalzonen-B-Zellen, B-1-Zellen, B-2-Zellen, regulatorische B-Zellen oder eine Kombination davon handelt, oder wobei es sich bei den T-Zellen um T-Helferzellen, zytotoxische T-Zellen, Gedächtnis-T-Zellen, Suppressor-T-Zellen, natürliche Killer T-Zellen, Mukosa-assoziierte invariante T-Zellen, gamma-delta-T-Zellen oder eine Kombination davon handelt.

15. Verbindung zur Verwendung nach einem der vorhergehenden Ansprüche, wobei eine Anregung des Immungedächtnisses durch die Bildung eines Nukleinsäureaddukts aktiviert wird, wobei es sich beim Nukleinsäureaddukt gegebenenfalls um ein doppelsträngiges DNA-Addukt, ein einsträngiges DNA-Addukt, ein doppelsträngiges RNA-Addukt oder ein einsträngiges RNA-Addukt handelt.

## Revendications

1. Composé de formule I ou II, ou sel correspondant, pour une utilisation dans un procédé d'induction d'une mémoire immunitaire chez un sujet atteint d'un cancer, comprenant une administration d'une quantité d'un composé de formule I ou II, ou d'un sel correspondant, au sujet : 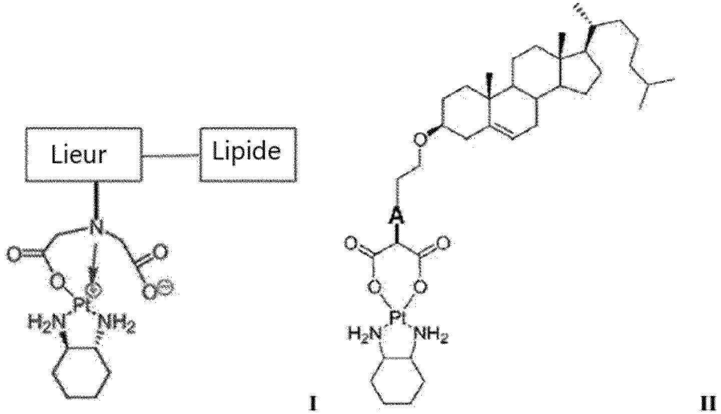
A étant absent ou cyclobutyle ;
le lipide étant un stérol ; et
le lieur étant -CH₂CH₂-, -CH₂CH₂NHC(O)-, -CH₂C(O)NHCH₂CH₂-, - CH₂CH₂OCH₂CH₂-, -C(O)CH₂-, ou -CH₂CH₂NHC(O)CH₂-.

2. Composé pour une utilisation selon la revendication 1, le composé induisant une mémoire immunitaire médiée par des cellules B chez le sujet.

3. Composé pour une utilisation selon l'une quelconque des revendications précédentes, le composé étant de formule (I) :

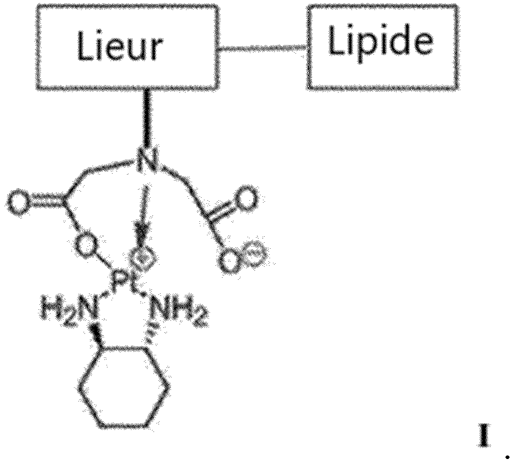

4. Composé pour une utilisation selon l'une quelconque des revendications précédentes, le lipide étant le lumistérol, le cholestérol ou le chloroformiate de cholestérol.

5. Composé pour une utilisation selon la revendication 1 ou la revendication 2, le composé étant de formule (II) :

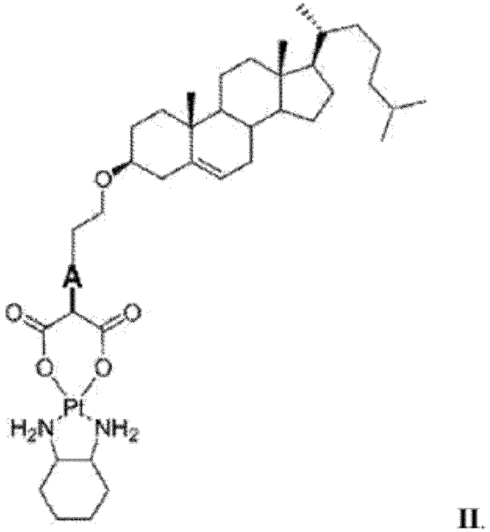

6. Composé pour une utilisation selon la revendication 1 ou la revendication 2, le composé étant : 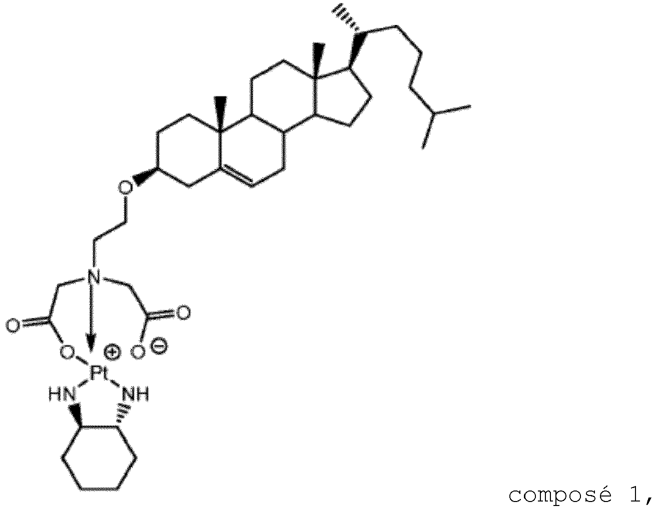 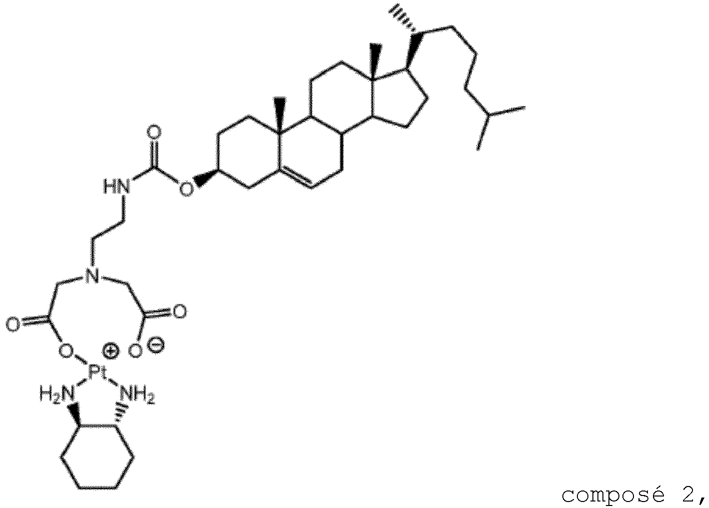 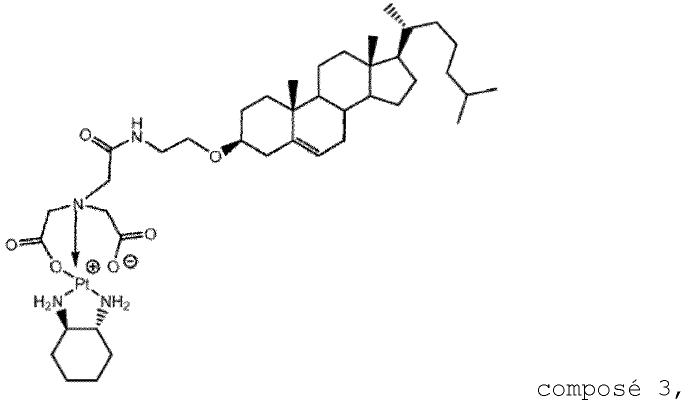 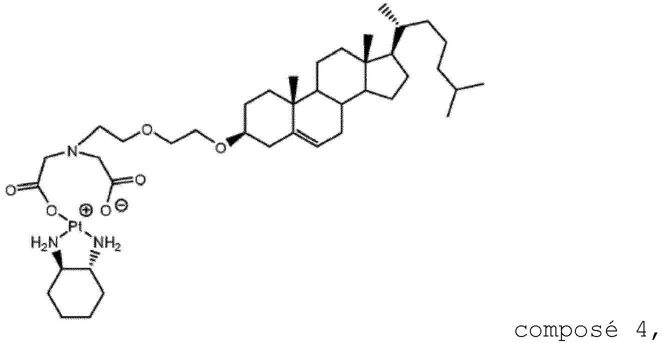 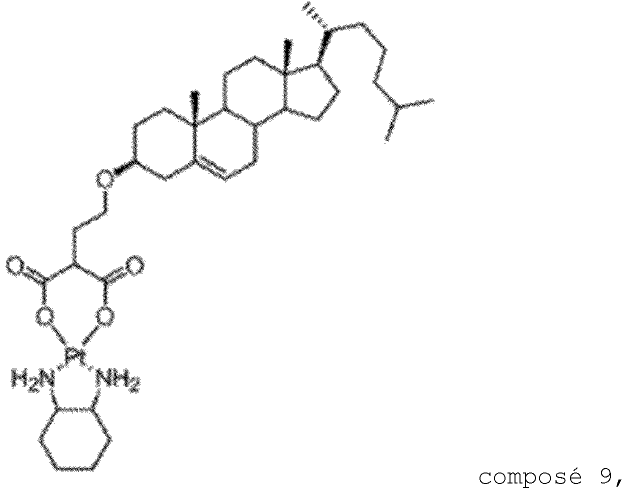 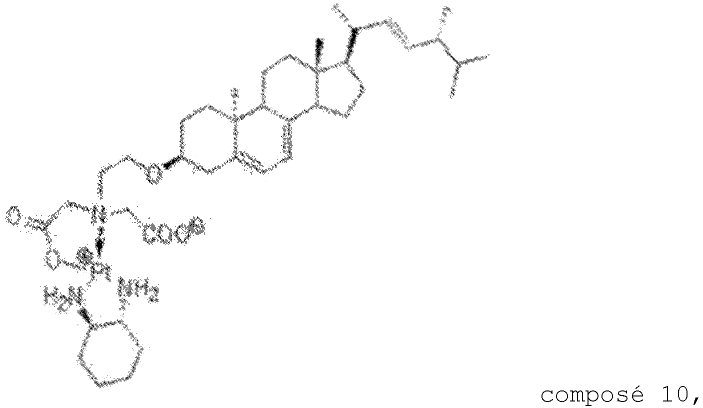 ou 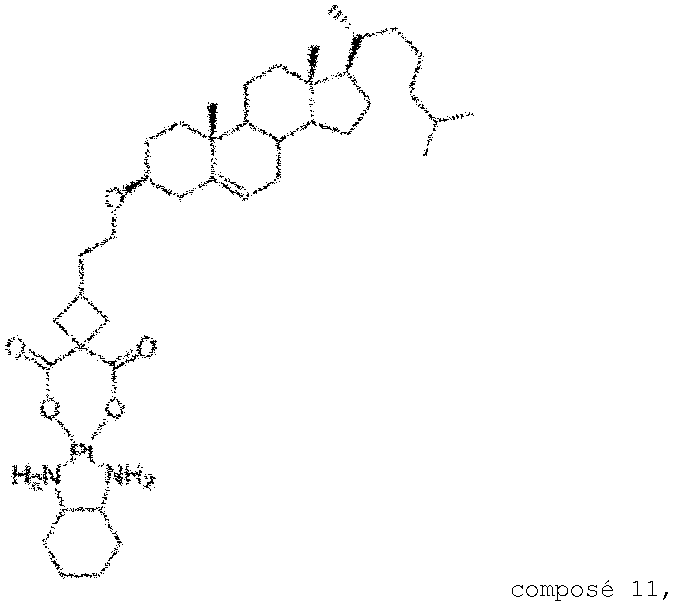 ou un sel correspondant.

7. Composé pour une utilisation selon la revendication 1 ou 2, le composé étant le composé 1 ou un sel correspondant :

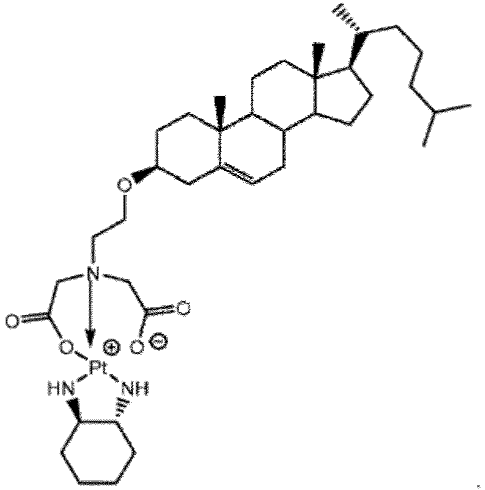

8. Composé pour une utilisation selon l'une quelconque des revendications 1 ou 3 à 7, le cancer étant un cancer du sein, un cancer de l'ovaire, un gliome, un cancer gastro-intestinal, un cancer de la prostate, un carcinome, un carcinome du poumon, un carcinome hépatocellulaire, un cancer testiculaire, un cancer du col de l'utérus, un cancer endométrial, un cancer de la vessie, un cancer de la tête et du cou, un cancer du poumon, un cancer gastro-œsophagien, un cancer gynécologique, ou une combinaison correspondante.

9. Composé pour une utilisation selon l'une quelconque des revendications précédentes, la quantité du composé fournissant une concentration en platine d'environ 50 mg/m² à environ 500 mg/m².

10. Composé pour une utilisation selon l'une quelconque des revendications précédentes, le composé étant administré par voie intraveineuse, par voie intra-articulaire, par voie artérielle duodénale pancréatique, par voie intrapéritonéale, par voie hépatoportale, par voie orale ou par voie intramusculaire.

11. Composé pour une utilisation selon l'une quelconque des revendications précédentes, le composé étant formulé dans une forme posologique qui est une injection, un comprimé, une poudre lyophilisée, une suspension liposomale, ou une combinaison correspondante.

12. Composé pour une utilisation selon l'une quelconque des revendications précédentes, l'administration augmentant l'expression d'immunoglobuline kappa C.

13. Composé pour une utilisation selon l'une quelconque des revendications précédentes, l'induction de la mémoire immunitaire étant médiée par le biais d'une ou plusieurs molécules immunopotentialisatrices, éventuellement la molécule immunopotentialisatrice activant des cytokines, des cellules B, des cellules T, des monocytes, des macrophages, des cellules tueuses naturelles, des cellules dendritiques, ou une combinaison correspondante.

14. Composé pour une utilisation selon la revendication 13, l'administration déclenchant une réponse humorale par le biais de cellules B, éventuellement les cellules B étant des plasmablastes, des plasmocytes, des cellules lymphoplasmacytoïdes, des cellules B à mémoire, des cellules B folliculaires, des cellules B de la zone marginale, des cellules B-1, des cellules B-2, des cellules B régulatrices, ou une combinaison correspondante ; ou les cellules T étant des cellules T auxiliaires, des cellules T cytotoxiques, des cellules T à mémoire, des cellules T suppressives, des cellules T tueuses naturelles, des cellules T invariantes associées aux muqueuses, des cellules T gamma delta, ou une combinaison correspondante.

15. Composé pour une utilisation selon l'une quelconque des revendications précédentes, l'induction de la mémoire immunitaire étant activée par la formation d'un adduit d'acide nucléique, éventuellement l'adduit d'acide nucléique étant un adduit d'ADN double brin, un adduit d'ADN simple brin, un adduit d'ARN double brin, ou un adduit d'ARN simple brin.
